# EUROPEAN PATENT APPLICATION

(11) **EP 3 640 319 A1**
(43) Date of publication of application: **22.04.2020**
(21) Application number: 18816577.3
(22) Date of filing: 26.04.2018
(51) Int. Cl.: C12M 1/34, C12M 3/00

(54) **INFORMATION PROCESSING DEVICE, INFORMATION PROCESSING SYSTEM, INFORMATION PROCESSING METHOD, AND PROGRAM**

(30) Priority: 13.06.2017 JP 2017115759
(71) Applicant: Sony Corporation, Tokyo 108-0075 (JP)
(72) Inventor: SHINODA, Masataka, Tokyo 108-0075 (JP)
(74) Representative: D Young & Co LLP
(86) International application number: PCT/JP2018/017004
(87) International publication number: WO 2018/230178

(57) **Abstract**

[Object] To provide an information processing apparatus, an information processing system, an information processing method, and a program which are suitable for assessing a fertile ovum with high precision.

[Solving Means] An information processing apparatus according to an embodiment of the present technology includes a storage unit, an image acquisition unit, and an assessment unit. The storage unit pre-stores a plurality of first images, the plurality of first images being obtained by rotating and imaging a comparative cell. The image acquisition unit acquires a plurality of second images, the plurality of second images being obtained by rotating and imaging a cell that is an object to be assessed. The assessment unit assesses the cell that is the object to be assessed on the basis of a result of comparison of the first image with the second image.

## Description

### Technical Field

The present technology relates to an information processing apparatus, an information processing system, an information processing method, and a program to be used for observing a culture cell.

### Background Art

In recent years, studies have been conducted in cell culture fields for fertility treatments, livestock breeding, and the like.

For example, for observing growth of a fertile ovum associated with in-vitro fertilization, the fertile ovum is put in a culture container including a storage portion that stores the fertile ovum. Then, the growth is observed. For observing a change over time of the growth of the fertile ovum, a camera images the fertile ovum to acquire an image (e.g., see Patent Literature 1). For the fertile ovum observation, it is desirable to assess the fertile ovum with high precision.

### Citation List

### Patent Literature

Patent Literature 1: Japanese Patent Application Laid-open No. 2011-192109

### Disclosure of Invention

### Technical Problem

When a taken image of a fertile ovum is used for assessing the fertile ovum, the shape of the fertile ovum after the blastocyst, for example, has high structural asymmetry. The look of the fertile ovum significantly thus differs depending on observation angles. Therefore, sufficient assessment cannot be performed only with an image obtained by imaging at a single observation angle.

It is an object of the present technology to provide an information processing apparatus, an information processing system, an information processing method, and a program which are suitable for assessing a fertile ovum with high precision.

### Solution to Problem

In order to accomplish the above-mentioned object, an information processing apparatus according to an embodiment of the present technology includes a storage unit, an image acquisition unit, and an assessment unit.

The storage unit pre-stores a plurality of first images, the plurality of first images being obtained by rotating and imaging a comparative cell.

The image acquisition unit acquires a plurality of second images, the plurality of second images being obtained by rotating and imaging a cell that is an object to be assessed.

The assessment unit assesses the cell that is the object to be assessed on the basis of a result of comparison of the first image with the second image.

With such a configuration, the comparative cell is rotated and imaged. Therefore, the storage unit stores a plurality of images (first images) of a fertile ovum imaged at different observation angles. Moreover, the cell that is the object to be assessed is rotated and imaged. Therefore, a plurality of images (second images) of a fertile ovum imaged at different observation angles can be obtained. The cell that is the object to be assessed is assessed by comparing the plurality of first images and the plurality of second images imaged at all the observation angles. Therefore, highly precise assessment can be performed.

The assessment unit may assess the cell that is the object to be assessed on the basis of a result of comparison of a first feature quantity extracted from the first image with a second feature quantity extracted from the second image.

The comparison can be performed by using the feature quantities extracted on the basis of the images in this manner. The feature quantity is information regarding a characteristic site of the image. The feature quantity includes size, shape, and sphericity of the fertile ovum, the number of times of cleavage (cleavage rate), forms of respective blastomeres and balance thereof, fragmentation, size and shape of an ICM, cell population and cell density of the ICM, and the like, for example.

The storage unit may store an assessment result of the first image which is associated with the first image. The information processing apparatus may further include: a determination unit that compares the first image with the second image and determines whether or not the first image is identical to the second image; and a giving unit that gives the second image the assessment result associated with the first image determined to be identical to the second image in a case where the determination unit determines that the first image is identical to the second image, in which the assessment unit considers a most given assessment result of assessment results respectively given to the plurality of second images as an assessment of the cell that is the object to be assessed.

In this manner, the assessment result associated with the first image identical to the second image can be considered as the assessment result of the second image and the most given assessment result of the assessment results respectively given to the plurality of second images can be considered as the assessment of the cell that is the object to be assessed.

The assessment result of the first image which is an image of a comparative fertile ovum is an observation by an assessment person such as an embryologist, for example, and includes an assessment result related to a growth stage of the fertile ovum and an assessment result related to quality of the fertile ovum, which have been determined by the assessment person. Therefore, the fertile ovum that is the object to be assessed can be precisely and easily assessed even without the presence of the assessment person. Further, the fertile ovum that is the object to be assessed can be assessed even without the presence of the assessment person. Therefore, it is effective for assessing numerous fertile ova.

The information processing apparatus may further include: a calculation unit that calculates an occupancy rate of the most given assessment result of the assessment results respectively given to the plurality of second images; and a determination control unit that determines whether or not a numerical value of the rate calculated by the calculation unit is stable and controls the determination unit to compare the first image with the second image until the determination control unit determines that the numerical value of the rate is stable.

In this manner, the comparing processing of the first image with the second image is performed until the numerical value of the rate of the most given assessment result of the assessment results respectively given to the plurality of second images becomes stable. Therefore, highly precise assessment can be performed.

The information processing apparatus may further include a rotation control unit that controls a rotation mechanism that rotates the cell.

The rotation control unit may control, in a case where the determination control unit determines that the numerical value of the rate is stable, the rotation mechanism to rotate the cell that is the object to be assessed.

Accordingly, images of the fertile ovum rotated under different rotation conditions can be acquired. Moreover, highly precise assessment can be performed.

The assessment unit may assess a growth stage of the cell that is the object to be assessed.

The assessment unit may assess quality of the cell that is the object to be assessed.

In this manner, the growth stage and the quality of the cell can be assessed.

An information processing system according to the present technology includes a culture container, an imaging unit, an image acquisition unit, a rotation mechanism, a storage unit, an image acquisition unit, and an assessment unit.

The culture container includes a plurality of storage portions in which a cell is stored.

The imaging unit images the cell.

The image acquisition unit acquires an image of the cell imaged by the imaging unit.

The rotation mechanism rotates the cell in one of the storage portions.

The storage unit pre-stores a first image acquired in such a manner that the imaging unit images the comparative cell rotated by the rotation mechanism.

The image acquisition unit acquires a plurality of second images, the plurality of second images being obtained in such a manner that the imaging unit images the cell that is an object to be assessed after the rotation mechanism rotates the cell that is the object to be assessed.

The assessment unit assesses the cell that is the object to be assessed on the basis of a result of comparison of the first image with the second image.

An information processing method according to the present technology includes: acquiring a plurality of second images by rotating and imaging a cell that is an object to be assessed; comparing a plurality of first images with each of the second images, the plurality of first images being acquired in advance by rotating and imaging a comparative cell; and assessing the cell that is the object to be assessed on the basis of the result of comparison.

A program according to the present technology causes a computer to execute: a step of acquiring a plurality of second images by rotating and imaging a cell that is an object to be assessed; a step of comparing a plurality of first images with each of the second images, the plurality of first images being acquired in advance by rotating and imaging a comparative cell; and a step of assessing the cell that is the object to be assessed on the basis of the result of comparison.

### Advantageous Effects of Invention

As described above, in accordance with the present technology, a fertile ovum that is an object to be assessed can be precisely assessed. It should be noted that the effects described here are not necessarily limitative and any effect described in the present disclosure may be provided.

### Brief Description of Drawings

[Fig. 1] A plan view of a culture container according to each embodiment.
[Fig. 2] An enlarged partial plan view of the culture container of Fig. 1.
[Fig. 3] A diagram describing a shape change caused by growth of a fertile ovum.
[Fig. 4] A schematic diagram of images of a fertile ovum on a growth stage after the blastocyst.
[Fig. 5] A schematic diagram showing a configuration of an observation system according to each of first to fourth embodiments.
[Fig. 6] A block diagram showing an observation system shown in Fig. 5.
[Fig. 7] A schematic plan view of a rotation apparatus that constitutes a part of the observation system shown in Fig. 5.
[Fig. 8] A schematic view of a vicinity of the rotation apparatus that constitutes the part of the observation system shown in Fig. 5.
[Fig. 9] A flowchart in generation of a learning database of the observation system shown in Fig. 5.
[Fig. 10] A diagram for describing driving timings of an observation illumination apparatus, a camera, and the rotation apparatus at the time of acquisition of a fertile ovum image in generation of the learning database of the observation system shown in Fig. 5.
[Fig. 11] A diagram for describing driving timings of vibration devices provided in the rotation apparatus in generation of the learning database of the observation system shown in Fig. 5 and assessment of fertile ova.
[Fig. 12] Diagrams each describing an example of a rotation pattern of fertile ova rotated by the rotation apparatus of the observation system shown in Fig. 5.
[Fig. 13] A diagram for describing another example of the driving timings of the observation illumination apparatus, the camera, and the rotation apparatus at the time of acquisition of a fertile ovum image in generation of the learning database of the observation system shown in Fig. 5.
[Fig. 14] A flowchart describing an assessment method for fertile ova that are objects to be assessed using the observation system shown in Fig. 5.
[Fig. 15] A diagram describing driving timings of the observation illumination apparatus, the camera, and the rotation apparatus at the time of acquisition of a fertile ovum image that is an object to be assessed using the observation system shown in Fig. 5.
[Fig. 16] A flowchart describing details of comparing, determining, and giving steps in the flowchart of Fig. 14.
[Fig. 17] A diagram describing another example of the driving timings of the observation illumination apparatus, the camera, and the rotation apparatus at the time of acquisition of a fertile ovum image that is an object to be assessed using the observation system shown in Fig. 5.
[Fig. 18] A diagram describing driving timings of the observation illumination apparatus, the camera, and the rotation apparatus at the time of acquisition of a fertile ovum image that is an object to be assessed using the observation system shown in Fig. 5 in a second embodiment.
[Fig. 19] A flowchart describing an assessment method for fertile ova using the observation system shown in Fig. 5 in a third embodiment.
[Fig. 20] A diagram describing a determination to terminate comparing processing in the third embodiment.
[Fig. 21] A flowchart describing details of image-acquiring, comparing, determining, and giving steps in the flowcharts of Figs. 19 and 22.
[Fig. 22] A flowchart describing an assessment method for fertile ova using the observation system shown in Fig. 5 in a fourth embodiment.
[Fig. 23] A diagram describing a determination to terminate comparing processing in the fourth embodiment.
[Fig. 24] A schematic diagram showing a configuration of an observation system of a fifth embodiment.
[Fig. 25] A block diagram showing an observation system according to the fifth embodiment.
[Fig. 26] An enlarged partial diagram of a culture container according to the fifth embodiment.
[Fig. 27] A diagram showing a configuration of a vicinity of a rotation unit of the observation system according to the fifth embodiment.
[Fig. 28] A schematic diagram showing a configuration of an observation system of the sixth embodiment. Mode(s) for Carrying Out the Invention

Hereinafter, embodiments according to the present technology will be described with reference to the drawings.

### <Configuration of Culture Vessel>

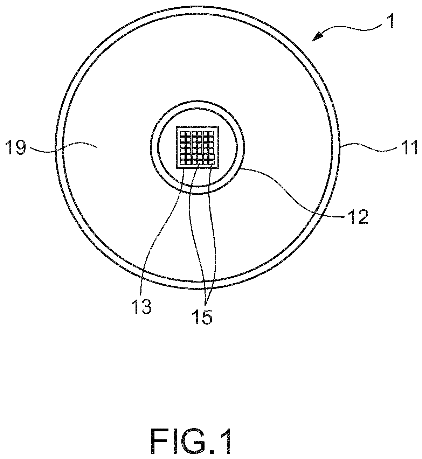

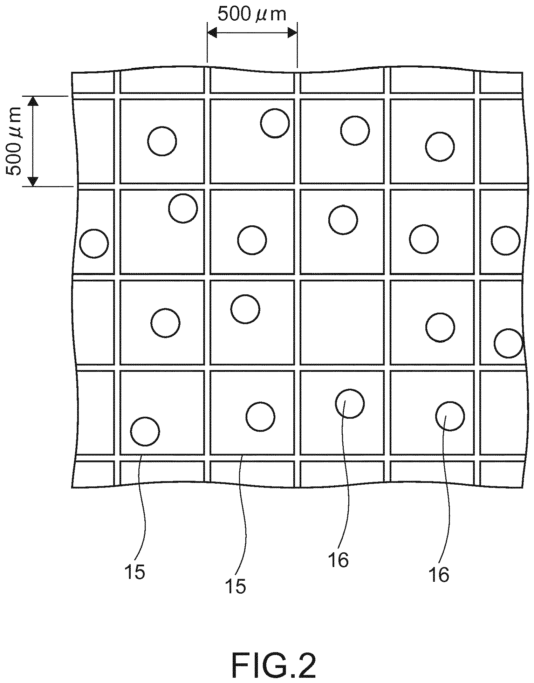

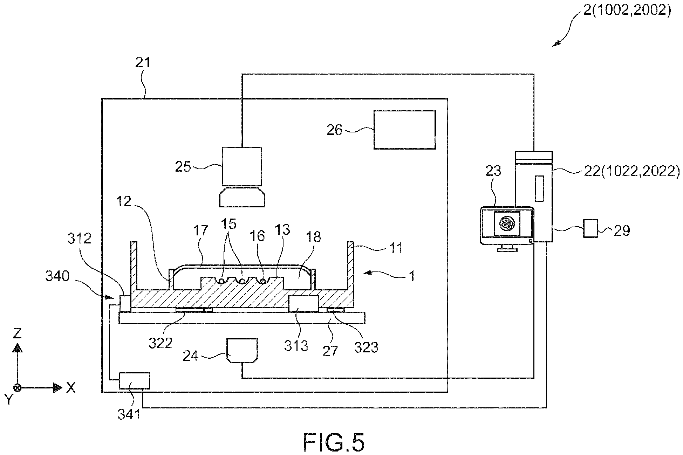

Fig. 1 is a plan view of a culture container (dish). Fig. 2 is an enlarged partial plan view of the culture container. Fig. 5 is a schematic diagram describing a state in which the culture container is housed inside an observation apparatus.

A culture container 1 is configured to be capable of storing a culture medium 18 and cells 16. The culture container 1 has a light transmittance such that the cells 16 can be imaged from the outside. It should be noted that there are no limitations on the number of culture containers 1 and the number of cells 16 which can be simultaneously imaged.

In this embodiment, fertile ova of creatures in the fields of livestock breeding and the like, for example, cows will be exemplified as the cells 16 to be cultured and described (hereinafter, shown as fertile ova 16 with the same reference sign). Not limited thereto, living body samples or the like taken out from a living body, such as stem cells, immune cells, and cancer cells in the fields of regenerative medicine and the like, for example, are exemplified as the cells to be cultured. The present technology is effective to cells which experience growth stages with shapes having high structural asymmetry and require three-dimensional images.

Moreover, in the present specification, the "cell" at least conceptually includes a single cell and an aggregate of a plurality of cells. Here, the single cell or the aggregate of the plurality of cells is related to a cell to be observed in one or more stages of growth processes of a fertile ovum, which includes an oocyte, an egg or ovum, a fertile ovum or zygote, a blastocyst, and an embryo (It should be noted that there are no limitations).

As shown in Fig. 1, the culture container 1 includes a bottom 19, an outer wall 11, an inner wall 12, storage portions 15, and a cell-arrangement convex portion 13.

The culture container 1 can be made of, for example, an inorganic material such as metal, glass, and silicon and an organic material such as a polystyrene resin, a polyethylene resin, a polypropylene resin, an ABS resin, nylon, an acrylic resin, a fluororesin, a polycarbonate resin, a polyurethane resin, a methylpentene resin, a phenol resin, a melamine resin, an epoxy resin, and a vinyl chloride resin. In this embodiment, the transparent culture container 1 made of polystyrene resin is used. In this embodiment, a case where the 36 storage portions 15 are arranged in one culture container 1 is exemplified and illustrated. However, the number of storage portions 15 is not limited thereto. It should be noted that the number of storage portions 15 of the culture container 1 in Fig. 5 is made different from the number of storage portions 15 of the culture container 1 in Fig. 1 for the sake of simplicity.

The plurality of storage portions 15 is provided. Each storage portion 15 is capable of storing a single cell, here, a fertile ovum 16 while maintaining the fertile ovum 16 at a fixed position. Further, each storage portion 15 stores liquid in addition to the fertile ovum 16. The "liquid" is typically a culture medium suitable for culturing cells. Hereinafter, the "liquid" will be referred to as a culture medium. As shown in Fig. 5, a culture medium 18 for culturing the fertile ova 16 is injected into the storage portions 15 and a region surrounded by the inner wall 12. In addition, in order to prevent evaporation of this culture medium 18, oil 17 is injected into the region surrounded by the inner wall 12 so as to cover the culture medium 18.

The bottom 19 has a circular plan shape. The outer wall 11 and the inner wall 12 are concentrically formed. The height of the inner wall 12 is set to be smaller than the height of the outer wall 11. The cell-arrangement convex portion 13 is arranged in the region surrounded by the inner wall 12 at the center of the bottom 19, at a distance from the inner wall 12. The cell-arrangement convex portion 13 has a rectangular plan shape. The 36 storage portions 15 are arranged in a matrix form, for example. A plan shape of the storage portion 15 is rectangular, though not limited thereto. The plan shape of the storage portion 15 may be circular.

<Shape Change Caused by Growth of Fertile Ovum>

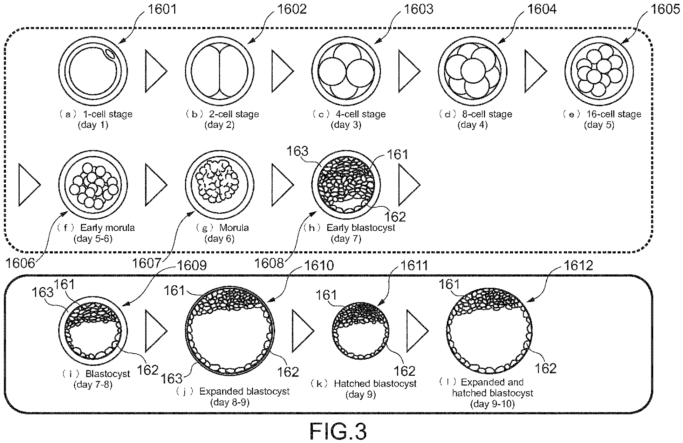

A shape change caused by growth of a fertile ovum will be described with reference to Fig. 3.

Fig. 3 shows a general growth stage of the fertile ovum 16 at day 1 to 10 after fertilization. Fig. 3(a) is a fertile ovum 1601 on the 1-cell stage at day 1 when fertilization is confirmed. At day 2 after fertilization, it is split into two and becomes a fertile ovum 1602 on the 2-cell stage as shown in Fig. 3(b). After that, it is normally grown up. The fertile ovum 16 becomes a fertile ovum 1603 on the 4-cell stage at day 3 after fertilization, a fertile ovum 1604 on the 8-cell stage at day 4 after fertilization, and a fertile ovum 1605 on the 16-cell stage at day 5 after fertilization in the stated order as shown in Fig. 3(c), Fig. 3(d), and Fig. 3(e). The cell population increases in this manner.

After that, the cells come close contact with each other. It becomes an early morula 1606 at day 5 to 6 after fertilization as shown in Fig. 3(f). It becomes a morula 1607 at day 6 after fertilization as shown in Fig. 3(g). Then, it is further grown up. A clearance is formed in cytoplasm and a blastocoel is formed. It becomes an early blastocyst 1608 at day 7 after fertilization as shown in Fig. 3(h). The blastocoel expands. Then, it becomes a blastocyst 1609 at day 7 to 8 after fertilization as shown in Fig. 3(i). On the growth stage (after 1608) of the blastocyst, an inner cell mass 161 (hereinafter referred to as ICM) which becomes an embryo in future can be identified from a trophectoderm 162 (hereinafter referred to as TE). On the growth stage of the early blastocyst 1608 and the blastocyst 1609, a zona pellucida 163 that forms an outer shape of the fertile ovum is recognized. Furthermore, the zona pellucida 163 becomes thinner. At day 8 to 9 after fertilization, the fertile ovum becomes an expanded blastocyst 1610. At day 9 after fertilization, it becomes a hatched blastocyst 1611, where the blastocyst departs from the zona pellucida. At day 9 to 10 after fertilization, it becomes an expanded and hatched blastocyst 1612.

The fertile ovum 16 has relatively high structural symmetry from fertilization to the growth stage of the morula 1607. The look does not significantly differ in a manner that depends on observation angles. For example, regarding the fertile ovum 16 on the growth stage of the 2-cell stage or the 4-cell stage, the look differs depending on observation angles. However, there are few blastomeres on those cell stages, and it is relatively easy to grasp a cleavage condition even by observation at a single observation angle, for example.

In contrast, once the blastocoel is formed and the fertile ovum 16 enters the growth stage (1608 to 1612) of the blastocyst, the structural asymmetry becomes higher and the look significantly differs depending on angles at which the fertile ovum 16 is viewed. As shown in Fig. 3(i), (j), (k), and (l), regarding the fertile ovum 16 on the growth stage after the blastocyst 1609, the occupancy rate of the blastocoel in the fertile ovum 16 increases. The ICM 161 is deviated from the center inside the spherical fertile ovum 16.

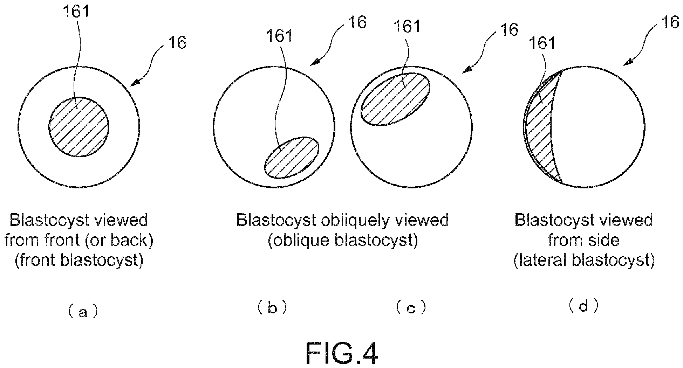

The look of the fertile ovum 16 significantly differs depending on observation angles on a certain growth stage. Fig. 4 is a schematic diagram of images obtained by imaging the fertile ovum 16 on a growth stage after the blastocyst 1609 at a plurality of angles.

Fig. 4(a) shows an image by imaging the fertile ovum 16 from the front or the back, provided that a state in which the ICM 161 is located at the center front of the fertile ovum 16 is referred to as the front. As shown in Fig. 4(a), it is an image in which the approximately circular ICM 161 is positioned at the center of the fertile ovum 16.

Fig. 4(d) shows an image obtained by imaging the fertile ovum 16 just beside the fertile ovum 16. As shown in Fig. 4(d), it is an image in which the ICM 161 having an approximately crescent shape is positioned at a side inside the fertile ovum 16.

Fig. 4(b) and (c) show image examples when the fertile ovum 16 is imaged in oblique directions, not just beside the fertile ovum 16 and from the back. As shown in Fig. 4(b) and (c), they are images each showing the ICM 161 positioned at a side inside the fertile ovum 16 and having an approximately elliptical shape.

As described above, the look of the fertile ovum 16 significantly differs depending on observation directions on a certain growth stage. An information processing system of the present technology is especially effective for assessing the fertile ovum on the growth stage on which the look of the fertile ovum significantly differs depending on such observation directions. Highly precise assessment can be performed.

Hereinafter, an observation system as the information processing system of the present technology will be described.

### <Overview of Observation System>

In the observation system as the information processing system of the present technology, data of a plurality of first images obtained by rotating and imaging comparative fertile ova on all growth stages is pre-stored in a learning database serving as a storage unit as learning data. Using the observation system in which this learning database has been generated, a plurality of second images obtained by rotating and imaging fertile ova that are objects to be assessed is acquired. In the observation system, each of the fertile ova that are the objects to be assessed is assessed by comparing information regarding those second images with information regarding the first images stored in the learning database.

Here, the images of the comparative fertile ova, which are the learning data, are referred to as first images. The images of the fertile ova that are the objects to be assessed are referred to as second images. A feature quantity extracted from each of the first images is referred to as a first feature quantity. A feature quantity extracted from each of the second images is referred to as a second feature quantity.

Further, the observation system including learning database in which the learning data has been stored is referred to as an observation system in assessment as necessary such that it is distinguished from the observation system in generation of the learning database.

The observation system includes a rotation mechanism. The rotation mechanism rotates the comparative fertile ova and the fertile ova that are the objects to be assessed.

In generation of the learning database, data of images obtained by rotating and imaging the fertile ova on each growth stage is acquired as the learning data. The learning data is stored in the learning database. This learning data stored in the learning database becomes comparative data in assessing the fertile ova that are the objects to be assessed.

The images (first images) of the fertile ova, which become the learning data, are obtained in such a manner that the rotation mechanism rotates the fertile ova and the camera serving as an imaging unit images the fertile ova. Images of the fertile ova imaged in a plurality of observation directions are acquired by rotating and imaging the fertile ova. A plurality of first images of a fertile ovum, which are acquired by imaging in the plurality of observation directions in a single image acquisition period, are associated with a first feature quantity, a growth stage code, a quality rank, and the like of that fertile ovum.
In this manner, the learning data is configured.

The feature quantity is information regarding a characteristic site of an image. The feature quantity includes size, shape, and sphericity of the fertile ovum, the number of times of cleavage (cleavage rate), forms of respective blastomeres and balance thereof, fragmentation, size and shape of the ICM, cell population and cell density of the ICM, and the like, for example.

The growth stage code and the quality rank are observations of the fertile ovum that an assessment person such as an embryologist have given by observing the image of that fertile ovum. The growth stage code and the quality rank are also an assessment result that the assessment person has given to the fertile ovum that is the object to be observed. The growth stage code indicates the growth stage of the fertile ovum. The quality rank indicates the quality of the fertile ovum.

Here, the growth stage includes the 1-cell stage, the 2-cell stage, the 4-cell stage, the 8-cell stage, the 16-cell stage, the early morula, the morula, the early blastocyst, the blastocyst, the expanded blastocyst, the hatched blastocyst, and the expanded and hatched blastocyst as described above.

For example, it is assumed that a growth stage of the 1-cell stage 1601 is a growth stage code 1, a growth stage of from the 2-cell stage 1602 to the 16-cell stage 1605 is a growth stage code 2, a growth stage of the early morula 1606 is a growth stage code 3, a growth stage of the morula 1607 is a growth stage code 4, a growth stage of the early blastocyst 1608 is a growth stage code 5, a growth stage of the blastocyst 1609 is a growth stage code 6, a growth stage of the expanded blastocyst 1610 is a growth stage code 7, a growth stage of the hatched blastocyst 1611 is a growth stage code 8, and a growth stage of the expanded and hatched blastocyst 1612 is a growth stage code 9. The growth stages are indicated by growth stage codes and stored in the learning database.

The quality rank is a result of quality assessment of the fertile ovum by the assessment person. The assessment criteria for quality assessment differ depending on fertile ovum growth stages. For example, on the 4 to 8-cell stages, the number of times of cleavage (cleavage rate) of the fertile ovum, the forms of blastomeres and balance thereof, the rate of fragmentation, and the like are classified into three ranks A to C as the assessment criteria. On the blastocyst stage, the cell population of the ICM, the cell density of the ICM, and the like are classified into the three ranks A to C as the assessment criteria. The quality is represented as a quality rank A to C and is stored in the learning database.

Each of the fertile ova that are the objects to be assessed is assessed using the learning data stored in the learning database of the observation system in assessment. In the observation system, a plurality of images (second images) of each of the fertile ova is acquired by rotating the fertile ova that are the objects to be assessed and imaging the rotated fertile ova in the plurality of observation directions.

Each of the acquired second images is compared with each of the first images. When it is determined in this comparing processing that the second image is identical to the first image, an assessment result associated with the first image determined to be identical to the second image is given to the second image used in the determination that they are identical. The assessment result includes the growth stage code (growth stage), the quality rank (quality), and the like.

Then, a most given assessment result of the assessment results respectively given to the plurality of second images is determined as an assessment with respect to the fertile ovum that is the object to be assessed.

A plurality of images (second images) of a fertile ovum that is an object to be assessed, which is obtained by imaging in the plurality of observation directions in the single image acquisition period, position information of the storage portion at which that fertile ovum is stored, date and time of imaging, an imaging condition, a second feature quantity, an assessment result of the fertile ovum that is the object to be assessed, and the like are associated with one another and stored in an analysis result database serving as a storage unit of the observation system.

### (First Embodiment)

### <Configuration of Observation System>

Hereinafter, an example of the observation system will be described.

The observation system that observes the fertile ova 16 stored in the culture container 1 will be described. It should be noted that in this embodiment, an incubator that cultures fertile ova of cows and an observation apparatus that observes the fertile ova are different apparatuses. Alternatively, a camera used for observing the fertile ova 16 may be arranged in the incubator to thereby provide a configuration in which the fertile ova can be observed in the incubator.

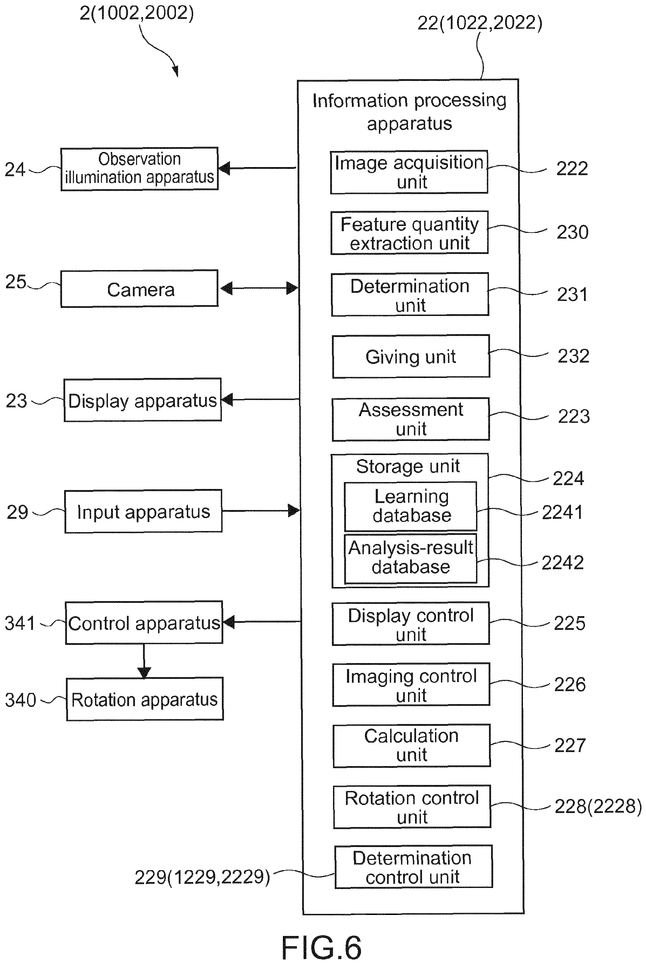

Fig. 5 is a schematic diagram showing the observation system. Fig. 6 is a block diagram showing a configuration of the observation system. In each of this embodiment and the embodiments to be described following this embodiment, an example in which one culture container is observed will be shown and described. Alternatively, the observation system may be configured to be capable of observing numerous fertile ova at a time by arranging one or more units each including a plurality of culture containers 1 set on a dish holder in the observation system.

As shown in Fig. 5, an observation system 2 includes an observation apparatus 21, an information processing apparatus 22, a displaying apparatus 23, an input apparatus 29, and a rotation apparatus 340.

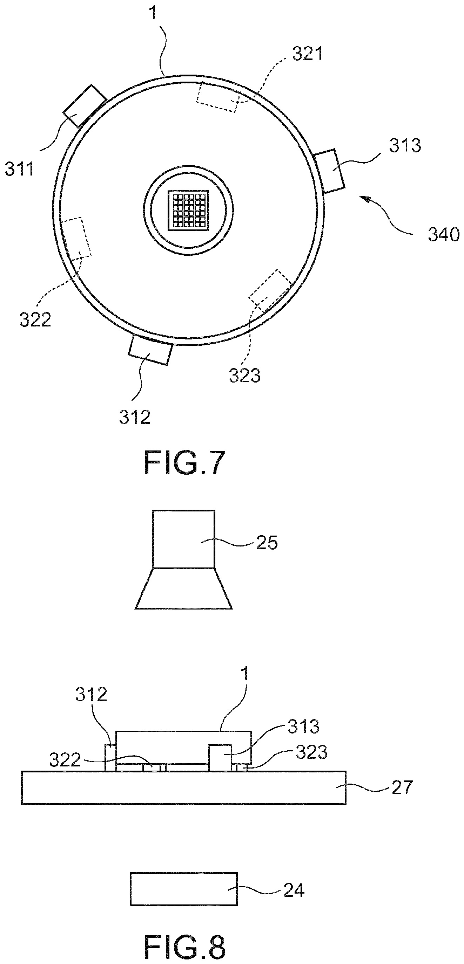

The rotation apparatus 340 serving as a rotation mechanism includes eccentric rotation motors (reference signs 311 to 313 and 321 to 323 shown in Fig. 7) and a control apparatus 341. The eccentric rotation motors serve as a plurality of vibrators. The control apparatus 341 receives a signal from the rotation control unit 228 to be described later and controls driving of each eccentric rotation motor.

The rotation apparatus 340 emits vibration and applies vibration on the culture container 1. The vibration of the culture container 1 rotates the fertile ova 16 stored in the storage portions 15 of the culture container 1. The rotation apparatus 340 is provided inside the observation apparatus 21. Details of the rotation apparatus 340 will be described later.

The observation apparatus 21 houses the culture container 1 in which the fertile ova 16 are stored and observes the fertile ova 16. The culture container 1 is horizontally held inside the observation apparatus 21. The fertile ova 16 are respectively stored in the storage portions 15 of the culture container 1 one by one. An observation illumination apparatus 24, a camera 25, a temperature/humidity/gas control unit 26, and a stage 27 are provided inside the observation apparatus 21.

The observation illumination apparatus 24 emits light to be radiated to the culture container 1 when the camera 25 images the fertile ova 16 in the culture container 1. Timings for turning on/off the light of the observation illumination apparatus 24 are controlled on the basis of imaging trigger signals from the imaging control unit 226 of the information processing apparatus 22 to be described later.

The camera 25 (hereinafter, shown as the camera 25 with the same reference sign) serving as an imaging unit includes, for example, a visible light camera including an image sensor such as a complementary metal-oxide semiconductor (CMOS) sensor and a charge coupled device (CCD) sensor. Instead of or in addition to the visible light camera, an infrared ray (IR) camera, a polarization camera, and the like may be used.

The camera 25 images the fertile ova 16 in the culture container 1. The camera 25 is provided inside the observation apparatus 21. The camera 25 includes a lens barrel including a lens group movable in an optical-axis direction (Z-axis direction), a solid-state image pickup device as an image pickup device such as a complementary metal oxide semiconductor (CMOS) and a charge coupled device (CCD) that captures object light passing the lens barrel, a driving circuit that drives them, and the like. It should be noted that the camera 25 may be installed inside the culture container 1 so as to be movable in the Z-axis direction and a horizontal plane direction (XY-plane direction) of the figure. Alternatively, the camera 25 may be configured to be capable of imaging not only a still image but also successive images (video).

The imaging control unit 226 of the information processing apparatus 22 to be described later controls the number of times of imaging, imaging timing, and the like of the camera 25.

The temperature/humidity/gas control unit 26 controls temperature, humidity, and gas inside the observation apparatus 21. The temperature/humidity/gas control unit 26 makes an environment suitable for culturing the fertile ova 16. The type of gas includes nitrogen, oxygen, carbon dioxide, and the like.

The input apparatus 29 is connected to the information processing apparatus 22. The input apparatus 29 is an operation device for inputting user's operations. A track ball, a touch pad, a mouse, a keyboard, and the like, for example, can be used as the input apparatus 29.

The displaying apparatus 23 outputs an image like a display. The displaying apparatus 23 displays an image of the fertile ovum 16, position information of the storage portion at which that fertile ovum 16 is stored, and information regarding date and time of imaging, a growth stage (growth stage code), quality (quality rank), and the like.

As shown in Fig. 6, the information processing apparatus 22 includes an image acquisition unit 222, a feature quantity extraction unit 230, a determination unit 231, a giving unit 232, an assessment unit 223, a storage unit 224, a display control unit 225, an imaging control unit 226, a calculation unit 227, a rotation control unit 228, and a determination control unit 229. The information processing apparatus 22 controls operations of the respective blocks of the observation system 2.

The information processing apparatus 22 includes hardware required for a computer configuration such as a central processing unit (CPU), a read only memory (ROM), a random access memory (RAM), and a hard disk drive (HDD). A personal computer (PC) is used as the information processing apparatus 22, for example. Alternatively, any other computer may be used.

The image acquisition unit 222, the feature quantity extraction unit 230, the determination unit 231, the giving unit 232, the assessment unit 223, the storage unit 224, the display control unit 225, the imaging control unit 226, the calculation unit 227, the rotation control unit 228, and the determination control unit 229 which are the functional blocks of the information processing apparatus 22 are realized in such a manner that the CPU loads a program stored in the ROM which is an example of a non-transitory computer-readable recording medium into the RAM and executes the loaded program. Then, those functional blocks execute an image acquisition method according to the present technology. The program is installed into the information processing apparatus 22 via various storage media, for example. Alternatively, the program may be installed via the Internet and the like.

The image acquisition unit 222 acquires from the camera 25 image information of the fertile ova imaged by the camera 25. Images of the fertile ova 16 imaged at a plurality of different angles can be acquired by imaging the fertile ova 16 a plurality of times while rotating the fertile ova 16 with an imaging angle fixed. It should be noted that here, the plurality of images are acquired while rotating the fertile ova 16. Alternatively, a moving image of the rotated fertile ova 16 may be acquired and a plurality of arbitrary images of the acquired moving image may be extracted.

First images and second images are both acquired in such a manner that the camera 25 images the fertile ova 16 rotated by the rotation apparatus 340.

On the basis of the image of the fertile ovum acquired by the image acquisition unit 222, the feature quantity extraction unit 230 extracts a feature quantity of that fertile ovum.

The feature quantity is information regarding a characteristic site of the image. The feature quantity information includes size, shape, and sphericity of the fertile ovum, the number of times of cleavage (cleavage rate), forms of respective blastomeres and balance thereof, fragmentation, size and shape of the ICM, cell population and cell density of the ICM, and the like, for example.

The storage unit 224 includes a learning database 2241 and an analysis-result database 2242. The learning database 2241 stores first images of comparative fertile ova and various types of data such as the first feature quantity as the learning data. The analysis-result database 2242 stores second images of fertile ova that are objects to be assessed and various types of data such as the second feature quantity.

In the observation system 2 in assessment, each of fertile ova that are objects to be assessed is assessed using the learning data stored in the learning database 2241.

In the observation system 2 in generation of the learning database, the storage unit 2241 serving as the learning database stores data in which first images obtained by rotating and imaging a single fertile ovum imaged in the single image acquisition period, a first feature quantity extracted by the feature quantity extraction unit 230, and a quality rank, a growth stage code, and the like are associated with one another. Data regarding fertile ova on all the growth stages of from the 1-cell stage to the expanded and hatched blastocyst is stored in the learning database 2241.

In the observation system 2 in assessment, the analysis-result database 2242 serving as the storage unit stores data in which a plurality of second images obtained by rotating and imaging a single fertile ovum that is an object to be assessed in the single image acquisition period, position information of the storage portion 15 at which the fertile ovum 16 that is the object to be assessed is stored, date and time of imaging, an imaging condition, a second feature quantity extracted by the feature quantity extraction unit 230, a growth stage code, a quality rank, and the like assessed by the assessment unit 223, are associated with one another. Such data is stored in the analysis-result database 2242 in time series for each storage portion 15.

In the observation system 2 in assessment, the determination unit 231 determines whether the first image is identical to the second image by comparing the first image pre-stored in the learning database 2241 of the storage unit 224 with the second image of the fertile ovum that is the object to be assessed.

More specifically, the determination unit 231 determines whether or not the second image which is the image of the fertile ovum 16 that is the object to be assessed is identical to the first image by comparing the first feature quantity extracted on the basis of the first image with the second feature quantity extracted on the basis of the second image. The determination result is input into the giving unit 232.

In the observation system 2 in assessment, the giving unit 232 gives a growth stage code and a quality rank to the fertile ovum 16 for assessment on the basis of the determination result of the determination unit 231.

In a case where the determination unit 231 determines that the first image is identical to the second image, the giving unit 232 gives the second image a growth stage code (growth stage) and a quality rank (quality assessment result) that are an assessment result associated with the first image determined to be identical to the second image. A single growth stage code and a single quality rank are given to a single second image.

The results given by the giving unit 232 are output to the calculation unit 227.

In the observation system 2 in assessment, the calculation unit 227 calculates, on the basis of the results given by the giving unit 232, an occupancy rate of each growth stage code to all growth stage codes respectively given to the plurality of second images. The calculation result calculated by the calculation unit 227 is output to the assessment unit 223.

Further, in the observation system 2 in assessment, the calculation unit 227 calculates, on the basis of the results given by the giving unit 232, an occupancy rate of each quality rank of the quality ranks respectively given to the plurality of second images. The calculation result calculated by the calculation unit 227 is output to the assessment unit 223.

In the observation system 2 in assessment, the assessment unit 223 assesses, on the basis of the input calculation result, a most given growth stage code (growth stage) of the growth stage codes (growth stages) respectively given to the plurality of second images of the fertile ovum 16 that is the object to be assessed as the growth stage code (growth stage) of the fertile ovum 16 for assessment.

Further, in the observation system 2 in assessment, the assessment unit 223 assesses, on the basis of the input calculation result, a most given quality rank of the quality ranks respectively given to the plurality of second images of the fertile ovum 16 that is the object to be assessed as the quality rank of the fertile ovum 16 for assessment.

It should be noted that in this embodiment, the case where both of the growth stage and the quality of the fertile ovum 16 that is the object to be assessed are assessed is exemplified and described. Alternatively, a configuration in which the assessment unit 223 assesses either the growth stage or the quality of the fertile ovum 16 may be employed.

In the observation system 2 in generation of the learning database, the display control unit 225 causes the displaying apparatus 23 to display the first images of the fertile ovum 16, an assessment entry field to which an input can be made by the assessment person, and the like.

In the observation system 2 in assessment, the display control unit 225 causes the displaying apparatus 23 to display the position information of the storage portion at which the fertile ovum 16 that is the object to be assessed is stored, the plurality of second images acquired by rotating that fertile ovum 16 which is the object to be assessed, the growth stage code (growth stage) or the quality rank (quality assessment result) assessed by the assessment unit 223, and the like.

The imaging control unit 226 outputs a control signal for controlling the number of times of imaging and the imaging timing of the fertile ovum 16 as the camera 25. The imaging control unit 226 generates an imaging trigger signal for performing imaging at predetermined time intervals. Note that, for example, control to reduce the number of times of imaging of the fertile ovum on the growth stage, which has high structural symmetry, and increase the number of times of imaging of the fertile ovum on the growth stage, which has high structural asymmetry, may be performed.

The rotation control unit 228 outputs a control signal for controlling a timing of driving/stopping and an applied voltage of each of the plurality of eccentric rotation motors provided in the rotation apparatus 340 to be described later, to the control apparatus 341. Accordingly, application/non-application of vibration to the culture container 1, the timing of application of vibration to the culture container 1, and the direction and the intensity of vibration are controlled.

By driving the eccentric rotation motors provided in the rotation apparatus 340, vibration is applied to the culture container 1. With this vibration application, the fertile ovum 16 stored in the culture container 1 is rotated. By adjusting the timing of driving/stopping each eccentric rotation motor and the applied voltage, the fertile ovum 16 can be rotated in a desired direction by an amount of rotation.

The rotation control unit 228 controls the timing of driving/stopping the rotation apparatus 340 on the basis of an imaging trigger signal from the imaging control unit 226.

Further, the rotation control unit 228 is capable of controlling the timing of driving/stopping each eccentric rotation motor and the applied voltage to control the direction of rotation and the amount of rotation of the fertile ovum 16.

For example, in the fertile ovum on the growth stage after the blastocyst, the shape and the position of the ICM in the fertile ovum 16 differ in a manner that depends on observation angles as shown in Fig. 4(a) to (d). In this embodiment, by adjusting the timing of driving/stopping each eccentric rotation motor and the applied voltage, the direction of rotation and the amount of rotation of the fertile ovum can be controlled such that the position and shape of the ICM become desired position and shape. The direction of rotation and the amount of rotation of the fertile ovum are controlled in the following manner.

On the basis of the image of the fertile ovum 16 imaged just before the fertile ovum 16 is rotated, the rotation control unit 228 detects the shape and position of the ICM 161 by image recognition such as edge detection. Based on it, the rotation control unit 228 calculates the direction of rotation and the amount of rotation of the fertile ovum 16 such that the image in which the ICM 161 shown in Fig. 4(a) is positioned at the center of the fertile ovum 16, for example, can be acquired. The rotation control unit 228 generates a control signal on the basis of this calculation result and sends the generated control signal to the control apparatus 341. The control apparatus 341 controls driving of each eccentric rotation motor of the rotation apparatus 340, such that the fertile ovum 16 rotates. Accordingly, the image in which the ICM is positioned at the center can be acquired.

In this manner, on the basis of the detection result of the shape and position of the ICM by image recognition, the direction of rotation and the amount of rotation of the fertile ovum 16 are calculated such that the image of the fertile ovum having desired shape and position of the ICM can be acquired. By rotating the fertile ovum 16 on the basis of it, a desired image can be acquired.

In the observation system 2 in assessment, the determination control unit 229 makes a determination to terminate the comparing processing of the first image with the second image. The determination to terminate the comparing processing may be arbitrarily set by the user. For example, the user sets a time and the number of first image compared with the second image. Alternatively, the time and the number of compared images, which lead to a determination to terminate the comparison, may be set in advance.

Fig. 7 is a schematic plan view of the rotation apparatus 340. Fig. 8 is a schematic partial view of the observation system 2 in vicinity of the rotation apparatus 340.

As shown in Figs. 7 and 8, the rotation apparatus 340 includes the eccentric rotation motors 311 to 313 and 321 to 323 as six vibration devices and the control apparatus 341. A piezoelectric element and the like may be used as the vibration devices in addition to the eccentric rotation motors.

The three eccentric rotation motors 321 to 323 are arranged at positions equally dividing an outer periphery of the culture container 1 whose outer plan shape is circular into three on an outer bottom surface of the culture container 1. The three eccentric rotation motors 311 to 313 are arranged at positions equally dividing the outer periphery into three on the outer side surface of the culture container 1. In a plan view of the culture container 1, along the outer periphery, the eccentric rotation motor 321 is positioned at a middle position between the eccentric rotation motor 311 and the eccentric rotation motor 313, the eccentric rotation motor 322 is positioned at a middle position between the eccentric rotation motor 311 and the eccentric rotation motor 312, and the eccentric rotation motor 323 is positioned at a middle position between the eccentric rotation motor 312 and the eccentric rotation motor 313.

The culture container 1 vibrates in such a manner that driving of each of the eccentric rotation motors 311 to 313 and 321 to 323 is controlled. With this vibration, the fertile ova 16 stored in the culture container 1 rotate.

By controlling a voltage applied to each of the eccentric rotation motors 311 to 313 and 321 to 323, the frequency (r.p.m. of motor) of vibration is controlled and the intensity of vibration is controlled. Further, by arranging the six eccentric rotation motors 311 to 313 and 321 to 32 at respective different positions, vibration in a desired direction can be applied to the culture container 1.

In this manner, by controlling driving/non-driving of each eccentric rotation motor, a driving voltage of driving of each eccentric rotation motor, and desired-intensity vibration in a desired direction can be applied to the culture container 1. Accordingly, the direction of rotation and the amount of rotation of the fertile ova 16 are controlled.

The culture container 1 equipped with the rotation apparatus 340 is placed on the stage 27. The fertile ova 16 stored in the culture container 1 can be imaged by the camera 25 arranged above the stage 27. Further, the observation illumination apparatus 24 is arranged below the stage 27. The observation illumination apparatus 24 radiates light to the fertile ova 16 in the culture container 1.

### <Generation of Learning Database of Observation System>

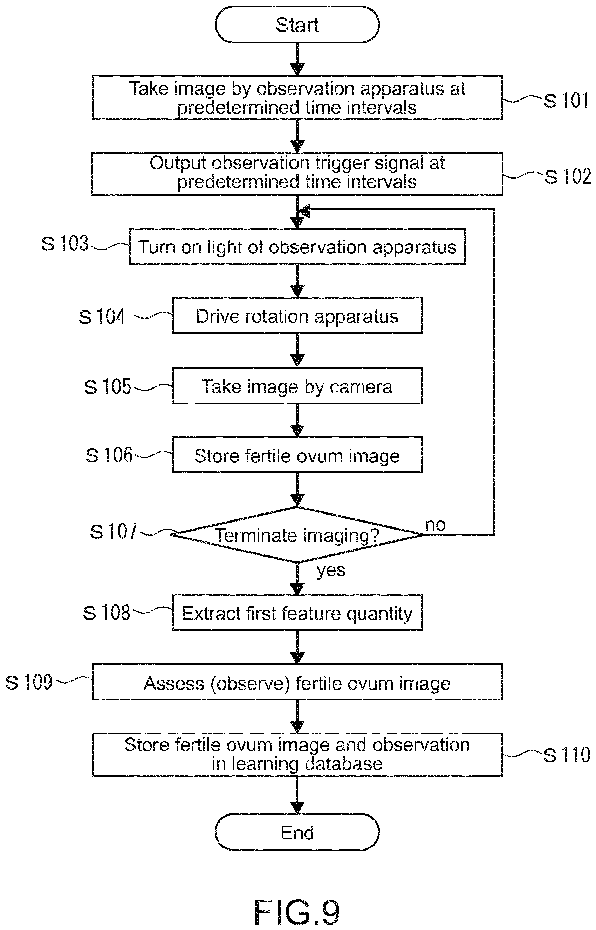

Next, generation of the learning database 2241 using the observation system 2 will be described with reference to Figs. 9 to 12. By rotating and imaging fertile ova on all the growth stages in generation of the learning database 2241, the images of the fertile ova imaged at the plurality of angles are acquired as the learning data.

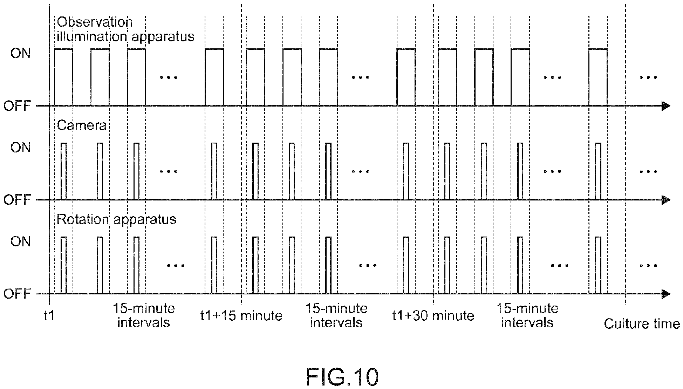

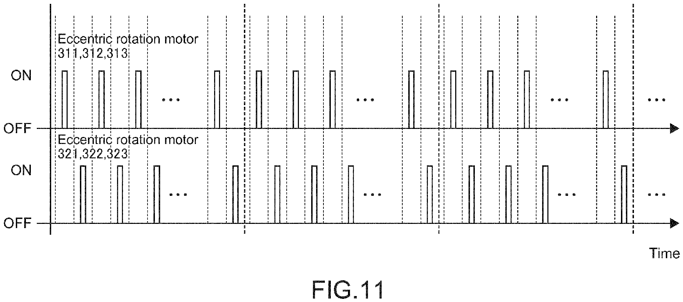

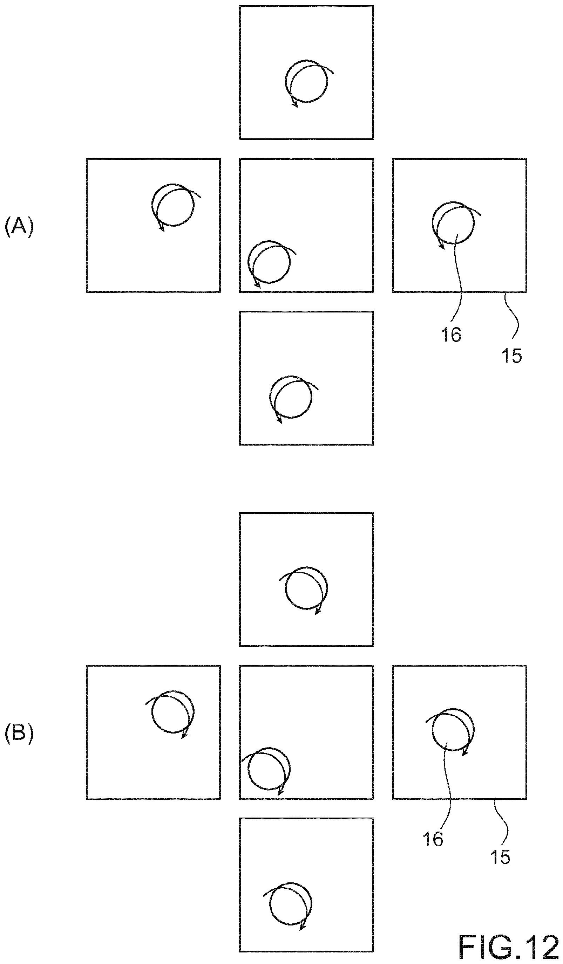

Fig. 9 is a flowchart of generation of the learning database 2241. Fig. 10 is a diagram for describing driving timings of the observation illumination apparatus, the camera, and the rotation apparatus at the time of acquisition of a fertile ovum image in generation of the learning database. Fig. 11 is a diagram describing driving timings of the eccentric rotation motors 311 to 313 and 321 to 323 provided in the rotation apparatus 340. Fig. 12 is a schematic view describing how the fertile ova 16 rotate by driving the rotation apparatus 340.

Image acquisition of the comparative fertile ova 16 is performed in a state in which the culture container 1 storing the fertile ova 16 is retained inside the observation apparatus 21. The image acquisition processing for the fertile ova 16 is performed in order for each of the fertile ova 16 respectively stored in the plurality of storage portions 15 of the culture container 1.

Regarding the image acquisition processing, data according to a plurality of first images acquired by imaging a single fertile ovum 16 in a single image acquisition period is stored in the database as one learning data item. Here, the single image acquisition period is set to be 15 minutes, for example. The learning data is data in which a first feature quantity of the fertile ovum, a growth stage code and a quality rank which are observations of an embryologist or the like with respect to the fertile ovum, and the first images of the fertile ovum imaged at a plurality of observation angles, which are acquired in the single image acquisition period, are associated with one another.

As shown in Fig. 10, the image acquisition of the fertile ova 16 is continuously performed in an n period. Here, the single image acquisition period is set to be 15 minutes. Light radiation of the observation illumination apparatus 24 is performed by repeating on/off in the single image acquisition period. The fertile ova 16 are intermittently irradiated with light. By intermittently radiating light in this manner, damage of the fertile ova due to light irradiation can be reduced. It should be noted that the time of the single image acquisition period is not limited to 15 minutes, and the time of the single image acquisition period can be arbitrarily set.

As shown in Fig. 10, imaging of the fertile ova 16 by the camera 25 is performed in the light radiation period of the observation illumination apparatus 24. Further, the rotation apparatus 340 drives in a state in which the observation illumination apparatus 24 and the camera 25 are both turned on. That is, the fertile ova 16 in the rotating states are imaged by the camera 25. The fertile ova 16 can be imaged at a plurality of different observation angles by taking a plurality of images of the fertile ova 16 rotating with the position of the camera 25 fixed.

Driving timings of the eccentric rotation motors 311 to 313 and 321 to 323 of the rotation apparatus 340 that rotate the fertile ova 16 will be described. As shown in Fig. 11, in this embodiment, driving of the eccentric rotation motors 311 to 313 arranged on the side surfaces of the culture container 1 and driving of the eccentric rotation motors 321 to 323 arranged on the bottom surface of the culture container 1 are alternately performed. It should be noted that timings of driving of the eccentric rotation motors 311 to 313 and driving of the eccentric rotation motors 321 to 323 are not limited thereto.

Examples of the driving condition of the rotation apparatus 340 and the number of times of imaging by the camera 25 will be shown.

The eccentric rotation motors whose rating r.p.m. is about 4000 rpm are used, only the eccentric rotation motors 321 to 323 are driven, and a direct-current voltage 1V is applied on the eccentric rotation motors 321 to 323. Under such a driving condition, the fertile ova 16 in the storage portions 15 rotate once for about one second. Those fertile ova 16 that rotate once for about one second is imaged by the camera 25 capable of 30-frame imaging for one second. Accordingly, 60 rotation images of the fertile ova 16 for two seconds can be acquired. Under this driving condition, for example, as shown in Fig. 12(A), the fertile ova 16 in the storage portions 15 all rotate in substantially the same direction of rotation by substantially the same amount of rotation.

Next, the eccentric rotation motors whose rating r.p.m. is about 4000 rpm are used, only the eccentric rotation motors 311 to 313 are driven, and a direct-current voltage 1V is applied on the eccentric rotation motors 311 to 313. Under such a driving condition, the fertile ova 16 in the storage portions 15 rotate once for about one second. Under this driving condition, for example, as shown in Fig. 12(B), the fertile ova 16 in the storage portions 15 all rotate in the substantially direction of rotation by substantially the same amount of rotation. By imaging those fertile ova 16 that rotate once for one second by the camera 25 capable of 30-frame imaging for one second in a manner that as described above, 60 rotation images of the fertile ova 16 for two seconds can be acquired.

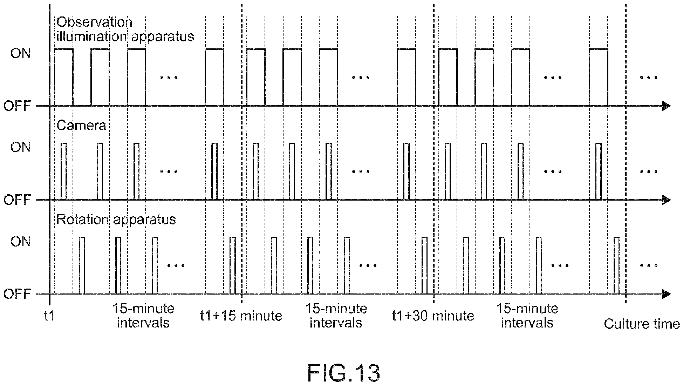

It should be noted that here, the camera 25 images the rotating fertile ova 16, though not limited thereto. The fertile ova 16 in a stationary state after rotation may be imaged as shown in Fig. 13 as another example.

As shown in Fig. 13, the single image acquisition period is set to be 15 minutes and the image acquisition of the fertile ova 16 is continuously performed for the n period. Light radiation of the observation illumination apparatus 24 is performed by repeating on/off. Light radiation is intermittently performed. By intermittently radiating light in this manner, damage of the fertile ova due to light irradiation can be reduced.

Imaging of the fertile ova 16 by the camera 25 is performed in the light radiation period of the observation illumination apparatus 24. Further, the rotation apparatus 340 is driven in a state in which the observation illumination apparatus 24 and the camera 25 are both turned off. That is, after the fertile ova 16 in the stationary states are imaged by the camera 25, imaging and rotation are repeatedly performed such that the rotation apparatus 340 is driven, the fertile ova 16 are rotated, and the fertile ova 16 in the stationary states after rotation are imaged, for example.

Hereinafter, a generation method of the learning database will be described following Fig. 9.

Fig. 9 shows processing performed in the single image acquisition period.

First of all, after the comparative fertile ova 16 confirmed to be fertilized are put in the storage portions 15 of the culture container 1 one by one, the culture medium 18 is injected into the storage portions 15 and into the region surrounded by the inner wall 12 with a pipette. After that, the oil 17 is injected into the region surrounded by the inner wall 12 so as to cover the culture medium 18.

Next, the culture container 1 is horizontally disposed on the stage 27 inside the observation apparatus 21 as shown in Fig. 5. At this time, as necessary, a transparent lid made of material similar to that of the culture container 1 (not shown) may be disposed on the culture container 1.

Next, imaging at predetermined time intervals is started (S101). Then, the imaging control unit 226 outputs an observation trigger signal (S102). Based on the observation trigger signal, illumination light of the observation illumination apparatus 24 is turned on at the timing shown in Fig. 10 (S103). The rotation apparatus 340 is driven (S104). The camera 25 performs imaging (S105).

The image acquisition unit 222 acquires a first image of each of the fertile ova 16 imaged by the camera 25 and the learning database 2241 stores the acquired first image (S106). Here, the image acquisition unit 222 may perform image pre-processing such as image normalization, position adjustment of the fertile ovum 16, and shape-emphasizing filtering on the acquired image of the fertile ovum 16. The recording unit 224 may store the first image on which this pre-processing has been performed.

The imaging control unit 226 determines whether or not to terminate imaging (S107). In this embodiment, 15 minutes are set as the single image acquisition period and the data corresponding to this single image acquisition period is stored as one data item. Therefore, when 15 minutes have elapsed from the imaging start, a determination to terminate imaging for the single image acquisition period is made.

In a case where 15 minutes have not elapsed and imaging for the single image acquisition period have not been terminated, the imaging control unit 226 makes a "no" determination at S107, returns to S103, and the steps of S103 to S106 are repeated.

In a case where 15 minutes have elapsed and imaging for the single image acquisition period has been terminated, the imaging control unit 226 makes a "yes" determination at S107 and proceeds to S108.

On the basis of each of the plurality of first images of the fertile ovum 16 for the single image acquisition period, which have been acquired by the image acquisition unit 222 and on which the pre-processing has been performed, the feature quantity extraction unit 230 extracts a first feature quantity of the fertile ovum 16 (S108).

An assessment person such as an embryologist sees the plurality of first image of the fertile ovum 16 imaged in the single image acquisition period, assesses the growth stage of the fertile ovum 16, and gives the growth stage code to the fertile ovum 16. Furthermore, the assessment person assesses the quality of the fertile ovum 16 on the basis of an assessment condition determined in advance for each growth stage and gives the quality rank to the fertile ovum 16.

In this manner, the assessment person assesses the fertile ovum 16 and gives the growth stage code and the quality rank as observations (S109).

Next, learning data in which the image of the single fertile ovum 16 imaged in the single image acquisition period, the first feature quantity of that fertile ovum 16, the growth stage code, and the quality rank are associated with one another is stored in the learning database 2241 (S110).

By repeating S101 to S110 described above, 15 minutes are set as the single image acquisition period and images of the comparative fertile ova 16 for the n period are acquired. Accordingly, the images (first images) of the comparative fertile ova 16 on all the growth stages which are imaged at the plurality of observation angles are acquired. Each of the first images of each of the comparative fertile ova imaged at the plurality of observation angles, which are imaged for each image acquisition period is stored in the learning database 2241 as the learning data such that a feature quantity (first feature quantity) of that image, a growth stage code, and a quality rank are associated with one another.

### <Assessment Method for Fertile Ova Using Observation System>

Next, an assessment method as an information processing method for fertile ova that are objects to be assessed using the observation system 2 in which the learning database has been generated as described above will be described.

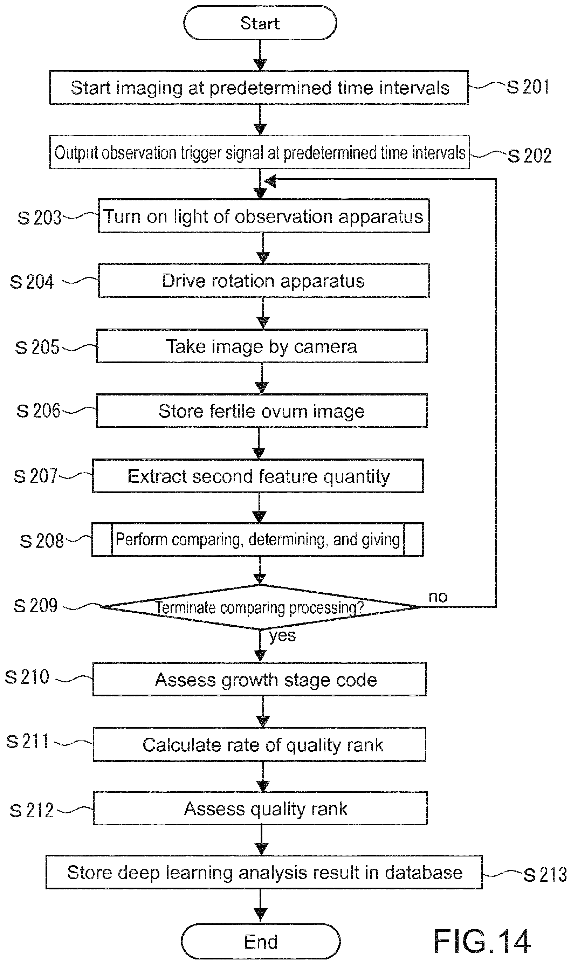

Hereinafter, the assessment method will be described with reference to Figs. 14 to 16.

Fig. 14 is a flowchart of an assessment method using the observation system 2 including the learning database 2241 in which the learning data has been stored.

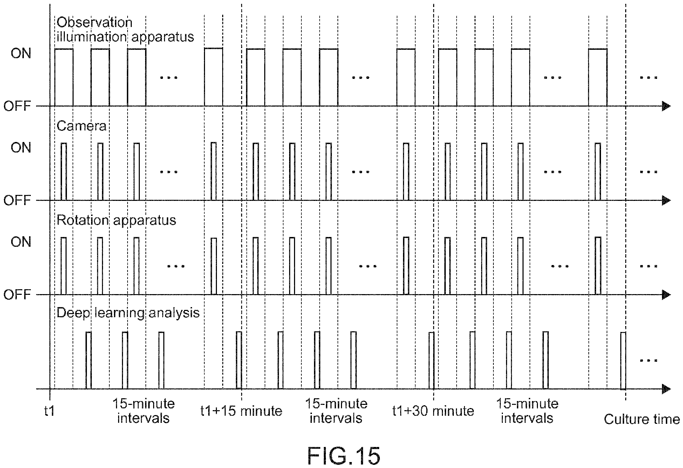

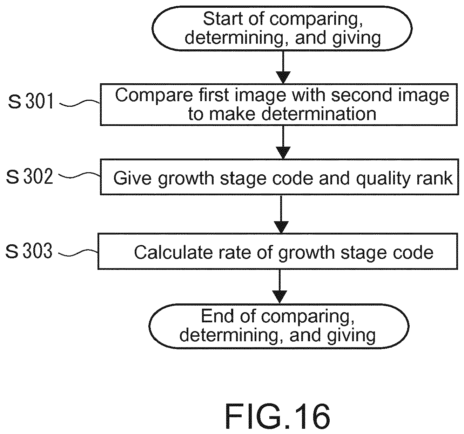

Fig. 15 is a diagram for describing driving timings of the observation illumination apparatus, the camera, and the rotation apparatus at the time of acquisition of a fertile ovum image using the observation system 2. Fig. 16 is a flowchart showing details of the comparing, determining, and giving steps of the fertile ovum image in the flowchart shown in Fig. 14.

In the image acquisition of the fertile ova 16 that are the objects to be assessed, the fertile ova 16 are rotated using the rotation apparatus 340 and the fertile ova 16 in the rotating states are imaged to thereby acquire images taken at the plurality of observation angles as in the image acquisition performed in generation of the learning database.

In this embodiment, as shown in Fig. 15, for example, 15 minutes are set as the single image acquisition period and the image acquisition processing is continuously performed for the n period as in generation of the learning database. Light radiation of the observation illumination apparatus 24 is performed by alternately repeating turning on/off of the light. Light radiation is intermittently performed. Also regarding imaging by the camera 25 and driving of the rotation apparatus 340, turning on/off is alternately repeated in accordance with turning on/off of the light of the observation illumination apparatus 24.

Imaging of the fertile ova 16 by the camera 25 is performed in the light radiation period of the observation illumination apparatus 24. Further, the rotation apparatus 340 is driven in a state in which the observation illumination apparatus 24 and the camera 25 are both turned on. That is, the fertile ova 16 in the rotating states are imaged by the camera 25.

Deep learning analysis of the second images of the fertile ova 16 that are the objects to be assessed, which are acquired by the camera 25, is performed when the observation illumination apparatus 24, the camera 25, and the rotation apparatus 340 are all turned off. The deep learning analysis is performed every time the camera 25 performs imaging. The deep learning analysis is performed a plurality of times in the single image acquisition period.

The driving timing of the eccentric rotation motors 311 to 313 and 321 to 323 of the rotation apparatus 340 which rotate the fertile ova 16 is similar to driving of the rotation apparatus 340 in generation of the learning database.

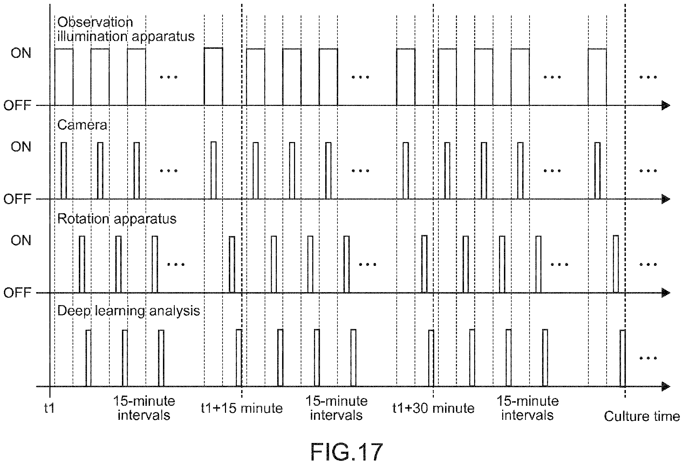

It should be noted that here, the camera 25 images the rotating fertile ova 16, though not limited thereto. As shown in Fig. 17, the rotation apparatus 340 may be driven in a state in which the observation illumination apparatus 24 and the camera 25 are both turned off and an image of the fertile ova 16 in the stationary states after rotation may be taken.

As shown in Fig. 17, the single image acquisition period is set to be 15 minutes and the image acquisition of the fertile ova 16 is continuously performed for the n period. Light radiation of the observation illumination apparatus 24 is performed by repeating on/off. Light radiation is intermittently performed.

As shown in Fig. 17, imaging of the fertile ova 16 by the camera 25 is performed in the light radiation period of the observation illumination apparatus 24. Further, the rotation apparatus 340 is driven in a state in which the observation illumination apparatus 24 and the camera 25 are both turned off. That is, after the camera 25 images the fertile ova 16 in the stationary states, imaging and rotation are repeatedly performed such that the rotation apparatus 340 is driven, the fertile ova 16 are rotated, and the fertile ova 16 in the stationary states after rotation are imaged, for example.

Further, the deep learning analysis is performed in the state in which the observation illumination apparatus 24 and the camera 25 are both turned off after driving of the rotation apparatus 340 is stopped.

Next, following Fig. 14, an assessment method for the fertile ova in the observation system 2 in assessment will be described.

Fig. 14 shows processing performed in the single image acquisition period.

First of all, after the fertile ova 16 confirmed to be fertilized are put in the storage portions 15 of the culture container 1 one by one, the culture medium 18 is injected into the storage portions 15 and into the region surrounded by the inner wall 12 with a pipette. After that, the oil 17 is injected into the region surrounded by the inner wall 12 so as to cover the culture medium 18.

Next, the culture container 1 is horizontally placed on the stage 27 inside the observation apparatus 21 as shown in Fig. 5. At this time, as necessary, a transparent lid made of a material similar to that of the culture container 1 (not shown) may be disposed on the culture container 1.

Next, when imaging is started at the predetermined time intervals (S201) and the imaging control unit 226 outputs an observation trigger signal (S202). Based on the observation trigger signal, illumination light of the observation illumination apparatus 24 is turned on at the timing shown in Fig. 15 (S203). The rotation apparatus 340 is driven (S204). The camera 25 performs imaging (S205).

The image acquisition unit 222 acquires the second images of the fertile ova 16 imaged by the camera 25. The acquired second images are stored in the analysis-result database 2242 as the storage unit (S206). Here, the image acquisition unit 222 performs image pre-processing such as image normalization, position adjustment of the fertile ova 16, and shape-emphasizing filtering on the second images of the acquired fertile ova 16. The analysis-result database 2242 may store the second images on which this pre-processing has been performed.

The feature quantity extraction unit 230 extracts a second feature quantity of the second image on the basis of the second images of the fertile ova 16 that are the objects to be assessed, which have been acquired by the image acquisition unit 222 and on which the pre-processing has been performed (S207).

Next, comparing the second images of the fertile ovum 16 that is the object to be assessed with the first images, determining, and giving assessment are performed (S208). The comparing, determining, and assessment-giving steps of will be described with reference to Fig. 16.

As shown in Fig. 16, the determination unit 231 compares the first image stored in the learning database 2241 with the second image stored in the analysis-result database 2242 and determines whether or not both are identical to each other. More specifically, the determination unit 231 compares the first feature quantity with the second feature quantity and determines whether or not the first image is identical to the second image (S301).

The giving unit 232 gives a growth stage code and a quality rank to the second image on the basis of the determination result of the determination unit 231 (S302). Specifically, in a case where the determination unit 231 determines that the first image is identical to the second image, the giving unit 232 gives the second image a growth stage code and a quality rank which are associated with the first image determined to be identical to the second image.

Such comparing, determining, and giving are performed for each second image and the growth stage code and the quality rank are given to each of the plurality of second images.

Next, the calculation unit 227 calculates an occupancy rate of each growth stage code of the growth stage codes respectively given to the plurality of second images by the giving unit 232 (S303). The calculation result calculated by the calculation unit 227 is output to the assessment unit 223.

Referring back to Fig. 14, the determination control unit 229 determines whether or not to terminate comparing processing of the first image with the second image (S209). In this embodiment, 15 minutes are set as the single image acquisition period and the data corresponding to this single image acquisition period is stored as one data item. Therefore, imaging for the single image acquisition period is terminated when 15 minutes have elapsed from the imaging start. In other words, a determination to terminate the comparing processing is made.

In a case where 15 minutes have not elapsed and imaging for the single image acquisition period have not been terminated, the determination control unit 229 determines to continue the comparing processing and a "no" determination is made at S209. When the "no" determination is made at S209, the processing returns to S203 and the steps of S203 to S208 are repeated.

In a case where 15 minutes have elapsed and imaging for the single image acquisition period has been terminated, the determination control unit 229 makes a "yes" determination at S209 and proceeds to S210.

It should be noted that in this embodiment, the determination to terminate the comparing processing is made on the basis of the time of the single image acquisition period, though not limited thereto. For example, the determination to terminate the comparing processing may be made on the basis of the number of first images compared with the second image. In this case, when comparison of the set number of images to be compared is completed before 15 minutes of the single image acquisition period set to be 15 minutes elapse, imaging of the fertile ova that are the objects to be assessed in that image acquisition period may be interrupted. When imaging is stopped, light radiation of the observation illumination apparatus is also stopped. Accordingly, the radiation time of light to the fertile ova 16 can be shortened, and the light influence on the fertile ova can be reduced.

In a case where it is determined to terminate the comparing processing at S209, the assessment unit 223 assesses, on the basis of the calculation result calculated by the calculation unit 227, a most given growth stage code of the growth stage codes respectively given to the plurality of second images as the growth stage code of the fertile ovum 16 that is the object to be assessed in the single image acquisition period (S210). The assessed growth stage code is given to the fertile ovum 16 that is the object to be assessed.

Next, the calculation unit 227 sets a second image given the growth stage code identical to the growth stage code given to the fertile ovum 16 that is the object to be assessed as an object and calculates a rate of the quality rank associated with the first image determined to be identical to the second image set as this object (S211). The calculation result calculated by the calculation unit 227 is output to the assessment unit 223.

The assessment unit 223 assesses, on the basis of the input calculation result, a most given quality rank of the quality ranks respectively given to a plurality of second images set as objects as the quality rank of the fertile ovum 16 for assessment (S212). The assessed quality rank is given to the fertile ovum 16 that is the object to be assessed.

In this manner, the second images of the fertile ovum 16 that is the object to be assessed imaged in the single image acquisition period at S208 to S212 are subjected to the deep learning analysis.

When the deep learning analysis of the second images of the fertile ovum 16 that is the object to be assessed is terminated, position information of the storage portion 15 at which the fertile ovum 16 that is the object to be assessed is stored, date and time of imaging, an imaging condition, the second feature quantity extracted by the feature quantity extraction unit 230, the plurality of images (second images) of the fertile ovum imaged in the plurality of observation directions, which are acquired by the image acquisition unit 222, the growth stage code and quality rank assessed by the assessment unit 223, and the like are associated with one another and are stored in the analysis-result database 2242 for each storage portion 15 (S213).

By repeating S201 to S213 described above, 15 minutes are set as the single image acquisition period and data of the fertile ova 16 that are the objects to be assessed for the n period is acquired. The second images acquired in each image acquisition period are stored in the analysis-result database 2242 in time series for each fertile ovum 16 while the second feature quantity, the position information of the storage portion 15, the date and time of imaging, the imaging condition, the growth stage code, the quality rank, and the like are associated with one another.

By using the observation system 2 in the above-mentioned manner, the growth stage and the quality of the fertile ovum 16 that is the object to be assessed can be correctly assessed on the basis of the learning data stored in the learning database 2241 without requiring observation by the assessment person. Highly precise assessment can be performed. Further, by using the observation system 2, the fertile ovum 16
that is the object to be assessed can be assessed without observation by the assessment person. Therefore, it is effective for assessing numerous fertile ova.

A specific example of the assessment method for the fertile ova 16 that are the objects to be assessed will be shown.

It is assumed that at S302, the giving unit 232 gives a growth stage code and a quality rank to each of 1000 second images.

For example, it is assumed that there are 30 second images given the growth stage code 4, 950 second images given the growth stage code 5, and 20 second images given the growth stage code 6. Then, at S303, the calculation unit 227 calculates an occupancy rate of the second images determined as the growth stage code 4 as 3%, an occupancy rate of the second images determined as the growth stage code 5 95%, and an occupancy rate of the second images determined as the growth stage code 6 as 2%.

At S210, the assessment unit 223 considers, on the basis of this calculation result, a most given growth stage code 5 of the growth stages given to the plurality of second images of the fertile ovum 16 that is the object to be assessed as being the growth stage of the fertile ovum 16 for assessment.

Next, at S211, the calculation unit 227 sets the 950 second images given the growth stage code 5 which is the same as the assessment result given to the fertile ovum 16 that is the object to be assessed as an object and calculates an occupancy rate of each quality rank of the quality ranks respectively given to those second images. Provided that out of the 950 second images given the growth stage code 5, 893 second images are the rank A, 38 second images are the rank B, and 19 second images are the rank C, the calculation unit 227 calculates an occupancy rate of the rank A as 94%, an occupancy rate of the rank B as 4%, and an occupancy rate of the rank C as 2%.

Accordingly, at S212, the assessment unit 223 considers, on the basis of this calculation result, the most given quality rank A of the growth stages given to the second images given the growth stage code 5 of the fertile ovum 16 that is the object to be assessed as being the quality of the fertile ovum 16 for assessment.

As described above, in the present technology, the comparative fertile ova are rotated and image data of the fertile ova is acquired in generation of the learning database. Therefore, the data of the fertile ova as viewed at all observation angles can be acquired as the learning data.

Furthermore, in the observation system 2 in assessment, the fertile ova for assessment are rotated and the image data of the fertile ova is acquired. Therefore, the data of the fertile ova that are the objects to be assessed as viewed at all the observation angles can be acquired as data for assessment.

Then, the learning data including the image data of the fertile ovum as viewed at all the observation angles is used for assessing the image data of the fertile ovum as also viewed at all the observation angles. Therefore, the fertile ovum that is the object to be assessed can be assessed with high precision.

For example, in a case where the fertile ovum that is the object to be assessed is a fertile ovum after the growth stage after the blastocyst having high structural asymmetry and is also a fertile ovum whose look significantly differs in a manner that depends on observation angles, the growth stage and the quality of the fertile ovum that is the object to be assessed can be correctly assessed by using the observation system according to the present technology. The assessment precision is thus enhanced.

### (Second Embodiment)

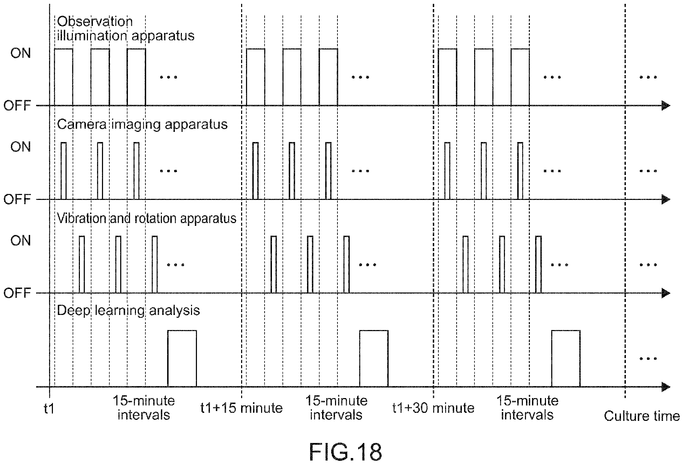

In accordance with the first embodiment, in the observation system in assessment, the deep learning analysis is performed every time the camera performs imaging, though not limited thereto. For example, the deep learning analysis may be performed once after the camera performs imaging a plurality of times in the single image acquisition period as shown in Fig. 18.

As shown in Fig. 18, the single image acquisition period is set to be 15 minutes and the image acquisition of the fertile ova 16 is continuously performed for the n period. Light radiation of the observation illumination apparatus 24 is performed by alternately repeating turning on/off of the light. Light radiation is intermittently performed. Also regarding imaging by the camera 25 and driving of the rotation apparatus 340, turning on/off is alternately repeated in accordance with turning on/off of the light of the observation illumination apparatus 24.

Imaging of the fertile ova 16 by the camera 25 is performed in the light radiation period of the observation illumination apparatus 24. Further, the rotation apparatus 340 is driven in a state in which the observation illumination apparatus 24 and the camera 25 are both turned on. That is, the fertile ova 16 in the rotating states are imaged by the camera 25.

Deep learning analysis of the second images of the fertile ova 16 that are the objects to be assessed, which are acquired by the camera 25, is performed when the observation illumination apparatus 24, the camera 25, and the rotation apparatus 340 are all turned off. In this embodiment, the second images acquired by the camera performing imaging a plurality of times in the single image acquisition period are analyzed at a time in single deep learning analysis.

### (Third Embodiment)

In the first embodiment, the determination to terminate the comparing processing of the first image with the second image is made on the basis of the time, the number of compared images, and the like set by the user in advance, for example, though not limited thereto. Hereinafter, another example of the determination to terminate the comparing processing will be described with reference to Figs. 6 and Figs. 19 to 21. Configurations similar to those of the embodiments will be denoted by similar reference signs and descriptions will be omitted. Moreover, here, an example in which the fertile ova in the rotating states are imaged will be shown and described.

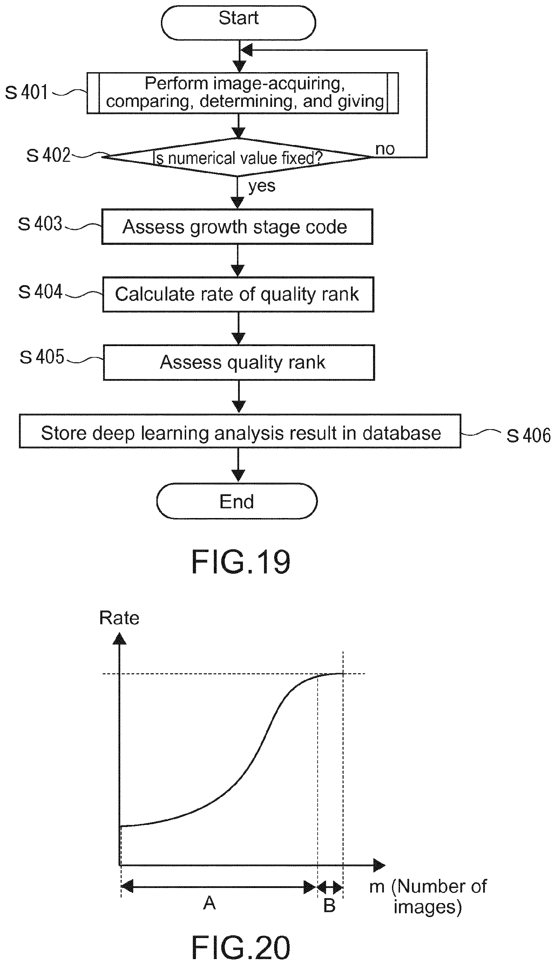

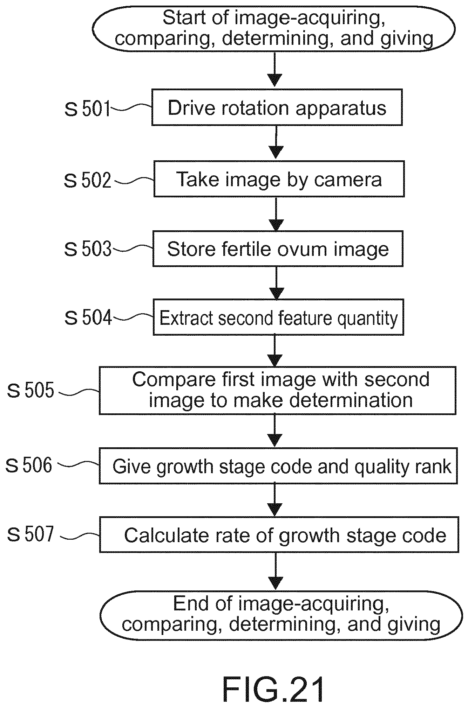

Fig. 6 is a block diagram showing a configuration of the observation system in this embodiment. Fig. 19 is a flowchart describing an assessment method for fertile ova using the observation system in assessment in this embodiment. Fig. 20 is a diagram describing the determination to terminate the comparing processing. Fig. 21 is a flowchart describing details of image-acquiring, comparing, determining, and giving steps in the flowchart of Fig. 19.

As shown in Fig. 6, an information processing apparatus 1022 of an observation system 1002 includes the image acquisition unit 222, the feature quantity extraction unit 230, the determination unit 231, the giving unit 232, the assessment unit 223, the storage unit 224, the display control unit 225, the imaging control unit 226, the calculation unit 227, the rotation control unit 228, a determination control unit 1229.

The determination control unit 1229 makes a determination to terminate the comparing processing of the first image with the second image. The determination control unit 1229 determines whether or not a numerical value of an occupancy rate of a most given growth stage of the growth stages respectively given to the second images, which is calculated by the calculation unit 227, is stable. The determination control unit 1229 controls the determination unit 231 to compare the first image with the second image until the determination control unit determines that the numerical value of the rate is stable.

The determination control unit 1229 determines that the numerical value of the rate is stable and controls the determination unit 231 to terminate the comparing processing.

When the determination control unit 1229 determines that the numerical value of the rate fluctuates and is instable, the determination control unit 1229 controls the determination unit 231 to continue the comparing processing.

Following Fig. 19, an assessment method for the fertile ova 16 in this embodiment will be described. In Fig. 20, the horizontal axis indicates the number of images to be compared in comparison of the first image with the second image and the vertical axis indicates the occupancy rate of the most given growth stage.

Fig. 19 shows processing performed in the single image acquisition period.

In the deep learning analysis, as the number of images to be compared in comparison of the first image with the second image becomes larger, the analysis precision becomes higher.

First of all, as shown in Fig. 19, image-acquiring, comparing, determining, and giving are performed (S401). Specifically, as shown in Fig. 21, the rotation apparatus 340 is driven (S501) and the fertile ovum 16 that is the object to be assessed rotates. The rotating fertile ovum 16 is imaged by the camera 25 (S502). The second image of the fertile ovum 16 imaged is stored in the analysis-result database 2242 (S503).

The feature quantity extraction unit 230 extracts a second feature quantity on the basis of the second image of the fertile ovum 16 imaged (S504). The determination unit 231 compares the first feature quantity extracted on the basis of the first image stored in the learning database 2241 with the second feature quantity extracted on the basis of the second image stored in the analysis-result database 2242. The determination unit 231 compares the first feature quantity with the second feature quantity and determines whether the first image is identical to the second image (S505).

The giving unit 232 gives a growth stage code and a quality rank to the second image on the basis of the determination result of the determination unit 231 (S506). Specifically, in a case where the determination unit 231 determines that the first image is identical to the second image, the giving unit 232 gives the second image a growth stage code and a quality rank which are associated with the first image determined to be identical to the second image. Accordingly, the growth stage code and the quality rank are given to each of the plurality of second images.

Next, the calculation unit 227 calculates an occupancy rate of each growth stage code of the growth stage codes respectively given to the plurality of second images by the giving unit 232 (S507). The calculation result calculated by the calculation unit 227 is output to the determination control unit 1229.

Referring back to Fig. 19, the determination control unit 1229 determines whether or not the numerical value of the occupancy rate of the most given growth stage of the growth stages respectively given to the second images is stable(S402).

In a case where the numerical value of the occupancy rate of the most given growth stage of the growth stages respectively given to the second images, which is calculated by the calculation unit 227, fluctuates and the numerical value is instable as shown in a region A of Fig. 20, the determination control unit 1229 makes a determination to continue the comparing processing (no). In a case where the no determination is made at S402, returning to S401, the steps of S501 to S507 are repeated, and image-acquiring, comparing, determining, and giving are repeated.

On the other hand, in a case where the numerical value of the occupancy rate of the most given growth stage of the growth stages respectively given to the second images, which is calculated by the calculation unit 227, becomes stable as shown in a region B of Fig. 20, the determination control unit 1229 determines that the numerical value is stable and makes a determination to terminate the comparing processing (yes). In a case where the Yes determination is made at S402, the comparing processing ends and the processing shifts to S403.

In this embodiment, in a case where it is determined at S402 that the numerical value becomes stable before 15 minutes of the single image acquisition period set to be 15 minutes elapse, imaging of the fertile ovum that is the object to be assessed in the image acquisition period is interrupted. When imaging is stopped, light radiation of the observation illumination apparatus is also stopped. Accordingly, the radiation time of light to the fertile ova 16 can be shortened, and the light influence on the fertile ova can be reduced.

The assessment unit 223 determines a most given growth stage code of the growth stage codes respectively given to the plurality of second images as being the growth stage code of the fertile ovum 16 that is the object to be assessed on the basis of the calculation result calculated by the calculation unit 227 (S403). The assessed growth stage code is given to the fertile ovum 16 that is the object to be assessed.

Next, the calculation unit 227 sets the second image given a growth stage code identical to the determined growth stage code as an object and calculates a rate of the quality rank associated with the first image determined to be identical to the second image which is this object (S404). The calculation result calculated by the calculation unit 227 is output to the assessment unit 223.

The assessment unit 223 considers, on the basis of the input calculation result, a most given quality rank of the quality ranks respectively given to the plurality of second images of the fertile ovum 16 that is the object to be assessed as being the quality rank of the fertile ovum 16 for assessment (S405). The assessed quality rank is given to the fertile ovum 16 that is the object to be assessed.

As described above, the second images of the fertile ovum 16 that is the object to be assessed are subjected to the deep learning analysis in S401 to S405.

When the deep learning analysis of the second image of the fertile ovum 16 that is the object to be assessed is terminated, position information of the storage portion 15 at which the fertile ovum 16 that is the object to be assessed is stored, date and time of imaging, an imaging condition, the second feature quantity extracted by the feature quantity extraction unit 230, the plurality of images of the fertile ovum imaged in the plurality of observation directions, which have been acquired by the image acquisition unit 222 in the single image acquisition period, the growth stage code and quality rank assessed by the assessment unit 223, and the like are associated with one another and are stored in the analysis-result database 2242 for each storage portion 15 (S406).

As described above, the comparing processing may be configured to be terminated in a case where the numerical value of the occupancy rate of the most given growth stage of the growth stages respectively given to the second images becomes stable. Accordingly, more highly precise assessment can be performed.

### (Fourth Embodiment)

In the first embodiment, the determination to terminate the comparing processing is made on the basis of the time, the number of compared images, and the like set by the user in advance, for example, though not limited thereto. Hereinafter, still another example of the determination to terminate the comparing processing will be described with reference to Fig. 6 and Figs. 21 to 23. Configurations similar to those of the embodiments will be denoted by similar reference signs and descriptions will be omitted. Moreover, here, an example in which the fertile ova in the rotating states are imaged will be shown and described.

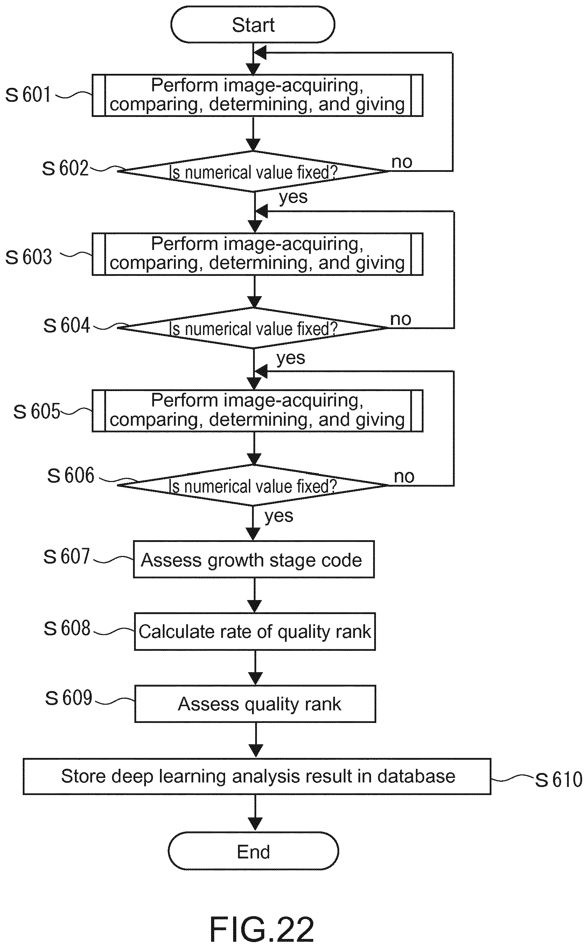

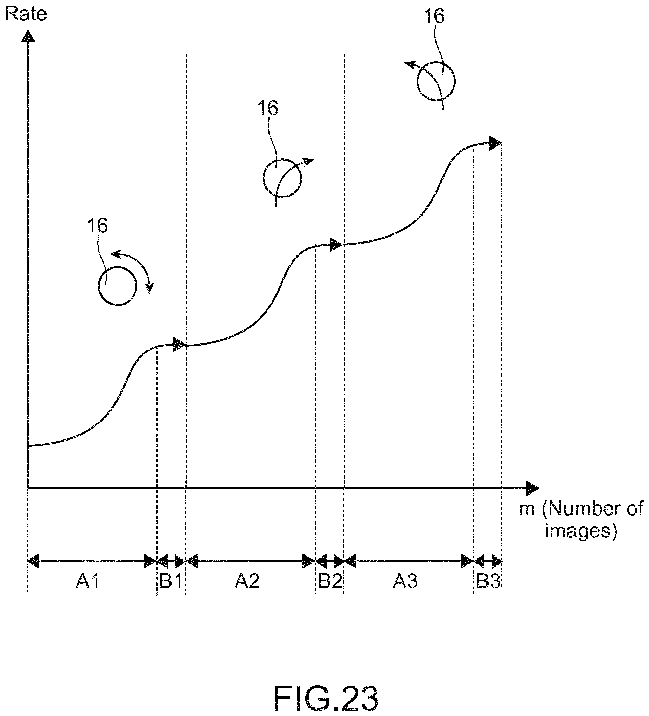

Fig. 6 is a block diagram showing a configuration of the observation system in this embodiment. Fig. 22 is a flowchart describing an assessment method for fertile ova using the observation system in this embodiment. Fig. 23 is a diagram describing a determination to terminate the comparing processing. Fig. 21 is a flowchart describing details of image-acquiring, comparing, determining, and giving steps in the flowchart of Fig. 22.

As shown in Fig. 6, an information processing apparatus 2022 of an observation system 2002 includes the image acquisition unit 222, the feature quantity extraction unit 230, the determination unit 231, the giving unit 232, the assessment unit 223, the storage unit 224, the display control unit 225, the imaging control unit 226, the calculation unit 227, a rotation control unit 2228, and a determination control unit 2229.

The determination control unit 2229 makes a determination to terminate the comparing processing of the second image with each of the first images acquired by imaging the fertile ova rotated under a certain rotation condition. The determination control unit 2229 determines whether or not the numerical value of the occupancy rate of the most given growth stage of the growth stages respectively given to the second images, which is calculated by the calculation unit 227, is stable. The determination control unit 2229 controls the determination unit 231 to compare the first image with the second image until the determination control unit determines that the numerical value of the rate is stable.

In a case where the determination control unit 2229 determines that the numerical value of the rate is stable, the determination control unit 2229 controls the determination unit 231 to terminate the comparing processing of the second image with each of the first images acquired by imaging the fertile ova rotated under the certain rotation condition.

In a case where the determination control unit 2229 determines that the numerical value of the rate fluctuates and is instable, the determination control unit 2229 makes a determination to continue the comparing processing.

Furthermore, in a case where the determination control unit 2229 makes a determination to terminate the comparing processing of the second image with each of the first images acquired by imaging the fertile ova rotated under the certain rotation condition, the determination control unit 2229 outputs a control signal to the rotation control unit 2228 to rotate the fertile ova 16 under a different rotation condition.

The rotation control unit 2228 receives the control signal from the determination control unit 2229 and outputs, to the control apparatus 341, a control signal for controlling the timing of driving/stopping each of the plurality of eccentric rotation motors provided in the rotation apparatus 340 and the applied voltage.

The rotation control unit 2228 is capable of controlling the direction of rotation and the amount of rotation of the fertile ova 16 as in the rotation control unit 228 of the first embodiment.

In this embodiment, as shown in Fig. 23, the rotation apparatus 340 is sequentially driven under three types of driving conditions and the fertile ovum 16 that is the object to be assessed is rotated under three types of different rotation conditions. A plurality of images of the fertile ovum 16 rotated under each of the different rotation conditions are acquired.

An assessment method for the fertile ova 16 in this embodiment will be described following Fig. 22. In Fig. 23, the horizontal axis indicates the number of images to be compared in comparison of the first image with the second image and the vertical axis indicates the occupancy rate of the most given growth stage.

First of all, as shown in Fig. 22, image-acquiring, comparing, determining, and giving are performed (S601). Specifically, as shown in Fig. 21, the rotation apparatus 340 is driven under a first driving condition (S501) and the fertile ovum 16 that is the object to be assessed is rotated under a first rotation condition. The rotating fertile ovum 16 is imaged by the camera 25 (S502). The second image of the fertile ovum 16 imaged is stored in the analysis-result database 2242 (S503).

The feature quantity extraction unit 230 extracts a second feature quantity on the basis of the second image of the fertile ovum 16 imaged (S504). The determination unit 231 compares the first feature quantity extracted on the basis of the first image stored in the learning database 2241 with the second feature quantity extracted on the basis of the second image stored in the analysis-result database 2242. The determination unit 231 compares the first feature quantity with the second feature quantity and determines whether the first image is identical to the second image (S505).

The giving unit 232 gives a growth stage code and a quality rank to the second image on the basis of the determination result of the determination unit 231 (S506). Specifically, in a case where the determination unit 231 determines that the first image is identical to the second image, the giving unit 232 gives the second image a growth stage code and a quality rank which are associated with the first image determined to be identical to the second image. Accordingly, the growth stage code and the quality rank are given to each of the plurality of second images.

Next, the calculation unit 227 calculates an occupancy rate of each growth stage code of the growth stage codes respectively given to the plurality of second images by the giving unit 232 (S507). The calculation result calculated by the calculation unit 227 is output to the determination control unit 2229.

Referring back to Fig. 22, the determination control unit 2229 determines whether or not the numerical value of the occupancy rate of the most given growth stage of the growth stages respectively given to the second images is stable (S602).

In a case where the numerical value of the occupancy rate of the most given growth stage of the growth stages respectively given to the second images, which is calculated by the calculation unit 227, fluctuates as shown in a region A1 of Fig. 23, the determination control unit 2229 determines that the numerical value is instable and makes a determination to continue the comparing processing (no). In a case where the no determination is made at S602, returning to S601, the steps of S501 to S507 are repeated, and image-acquiring, comparing, determining, and giving are repeated.

On the other hand, in a case where the numerical value of the occupancy rate of the most given growth stage of the growth stages respectively given to the second images, which is calculated by the calculation unit 227, becomes stable as shown in a region B1 of Fig. 23, the determination control unit 2229 determines that the numerical value is stable and makes a determination to terminate the comparing processing (yes). In a case where the Yes determination is made at S602, the comparing processing ends and the processing shifts to S603.

In S603, image-acquiring, comparing, determining, and giving are performed. Specifically, as shown in Fig. 21, the rotation apparatus 340 is driven under a second driving condition (S501) and the fertile ovum 16 that is the object to be assessed is rotated under a second rotation condition. The second driving condition is different from the first driving condition. The second rotation condition is different from the first rotation condition.

Hereinafter, S502 to S507 are performed in a manner similar to that described above.

Referring back to Fig. 22, the determination control unit 2229 determines whether or not the numerical value of the occupancy rate of the most given growth stage of the growth stages respectively given to the second images is stable (S604).

In a case where the numerical value of the occupancy rate of the most given growth stage of the growth stages respectively given to the second images, which is calculated by the calculation unit 227, fluctuates as shown in a region A2 of Fig. 23, the determination control unit 2229 determines that the numerical value is instable and makes a determination to continue the comparing processing (no). In a case where the no determination is made at S604, returning to S603, the steps of S501 to S507 are repeated, and image-acquiring, comparing, determining, and giving are repeated.

On the other hand, in a case where the numerical value of the occupancy rate of the most given growth stage of the growth stages respectively given to the second images, which is calculated by the calculation unit 227, becomes stable as shown in a region B2 of Fig. 23, the determination control unit 2229 determines that the numerical value is stable and makes a determination to terminate the comparing processing (yes). In a case where the yes determination is made at S604, the comparing processing ends and the processing shifts to S605.

In S605, image-acquiring, comparing, determining, and giving are performed. Specifically, as shown in Fig. 21, the rotation apparatus 340 is driven under a third driving condition (S501). The fertile ovum 16 which is the object to be assessed rotates under a third rotation condition. The third driving condition is different from the first and second driving conditions. The third rotation condition is different from the first and second rotation conditions.

Hereinafter, S502 to S507 are performed in a manner similar to that described above.

Referring back to Fig. 22, the determination control unit 2229 determines whether or not the numerical value of the occupancy rate of the most given growth stage of the growth stages respectively given to the second images is stable (S606).

In a case where the numerical value of the occupancy rate of the most given growth stage of the growth stages respectively given to the second images, which is calculated by the calculation unit 227, fluctuates as shown in a region A3 of Fig. 23, the determination control unit 2229 determines that the numerical value is instable and makes a determination to continue the comparing processing (no). In a case where the no determination is made at S606, returning to S605, the steps of S501 to S507 are repeated, and image-acquiring, comparing, determining, and giving are repeated.

On the other hand, in a case where the numerical value of the occupancy rate of the most given growth stage of the growth stages respectively given to the second images, which is calculated by the calculation unit 227, becomes stable as shown in a region B3 of Fig. 23, the determination control unit 2229 determines that the numerical value is stable and makes a determination to terminate the comparing processing (yes). In a case where the yes determination is made at S606, the comparing processing ends and the processing shifts to S607.

It should be noted that in this embodiment, the example in which the fertile ova 16 are rotated under three types of rotation conditions has been shown, though the types of rotation condition are not limited to three. The types of rotation condition can be arbitrarily set.

Further, in this embodiment, before 15 minutes of the single image acquisition period set to be 15 minutes elapse, in a case where it is determined at S606 that the numerical value is stable, imaging of the fertile ovum that is the object to be assessed in the image acquisition period is interrupted. When imaging is stopped, light radiation of the observation illumination apparatus is also stopped. Accordingly, the radiation time of light to the fertile ova 16 can be shortened, and the light influence on the fertile ova can be reduced.

The assessment unit 223 determines a most given growth stage code of the growth stage codes respectively given to the plurality of second images as being the growth stage code of the fertile ovum 16 that is the object to be assessed on the basis of the calculation result calculated by the calculation unit 227 (S607). The assessed growth stage code is given to the fertile ovum 16 that is the object to be assessed.

Next, the calculation unit 227 sets the second image given a growth stage code identical to the determined growth stage code as an object and calculates a rate of the quality rank associated with the first image determined to be identical to the second image which is this object (S608). The calculation result calculated by the calculation unit 227 is output to the assessment unit 223.

The assessment unit 223 considers, on the basis of the input calculation result, a most given quality rank of the quality ranks respectively given to the plurality of second images of the fertile ovum 16 that is the object to be assessed as being the quality rank of the fertile ovum 16 for assessment (S609). The assessed quality rank is given to the fertile ovum 16 that is the object to be assessed.

As described above, the second images of the fertile ovum 16 that is the object to be assessed are subjected to the deep learning analysis in S601 to S609.

In a case where the deep learning analysis of the second image of the fertile ovum 16 that is the object to be assessed is terminated, position information of the storage portion 15 at which the fertile ovum 16 that is the object to be assessed is stored, date and time of imaging, an imaging condition, the second feature quantity extracted by the feature quantity extraction unit 230, the plurality of images of the fertile ovum imaged in the plurality of observation directions, which have been acquired by the image acquisition unit 222 in the single image acquisition period, the growth stage code and quality rank assessed by the assessment unit 223, and the like are associated with one another and are stored in the analysis-result database 2242 for each storage portion 15 (S610).

As described above, the second images of the fertile ovum rotated under the varied rotation conditions may be acquired and the comparing processing may be configured to be terminated in a case where the numerical value of the occupancy rate of the most given growth stage of the growth stages given to the second images acquired under the respective rotation conditions becomes stable. Accordingly, more highly precise assessment can be performed.

### (Fifth Embodiment)

In each of the above-mentioned embodiments, the fertile ova 16 are rotated using the rotation apparatus that utilizes vibration. On the other hand, in this embodiment, the fertile ova 16 are rotated using a rotation apparatus that utilizes water flows. In this embodiment, fluid is ejected (injected) into the culture medium of the storage portions of the culture container. In this manner, a flow is generated in the culture medium in the storage portions to thereby rotate the fertile ova 16.

This embodiment is different from the above-mentioned embodiments mainly in that a culture container including a rotation mechanism that generates a flow in the culture medium and rotates the fertile ova, not a vibration apparatus. Configurations different from those of the above-mentioned embodiments will be mainly described. Configurations similar to those of the embodiments will be denoted by similar reference signs and descriptions will be omitted in some cases.

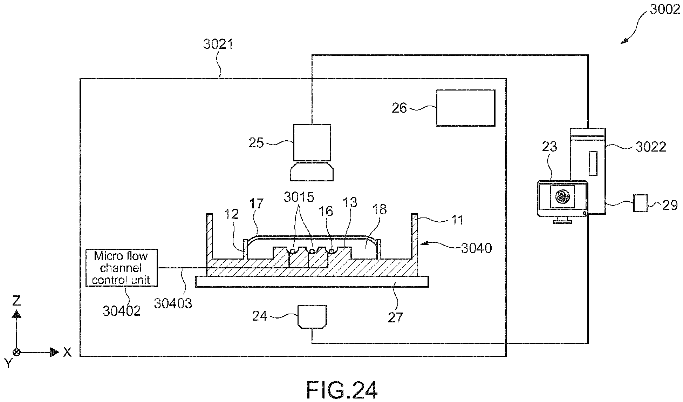

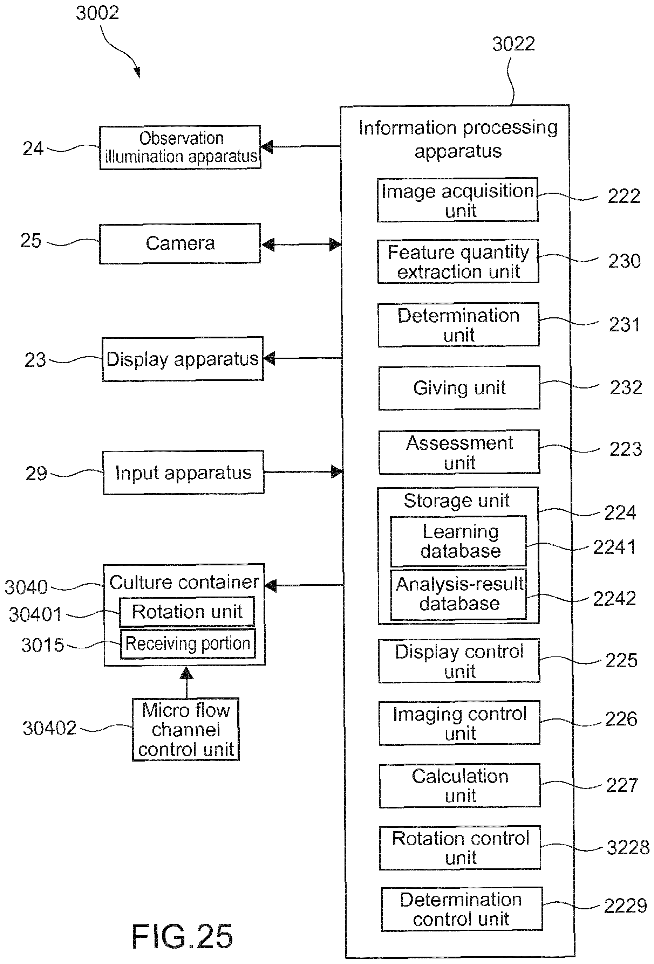

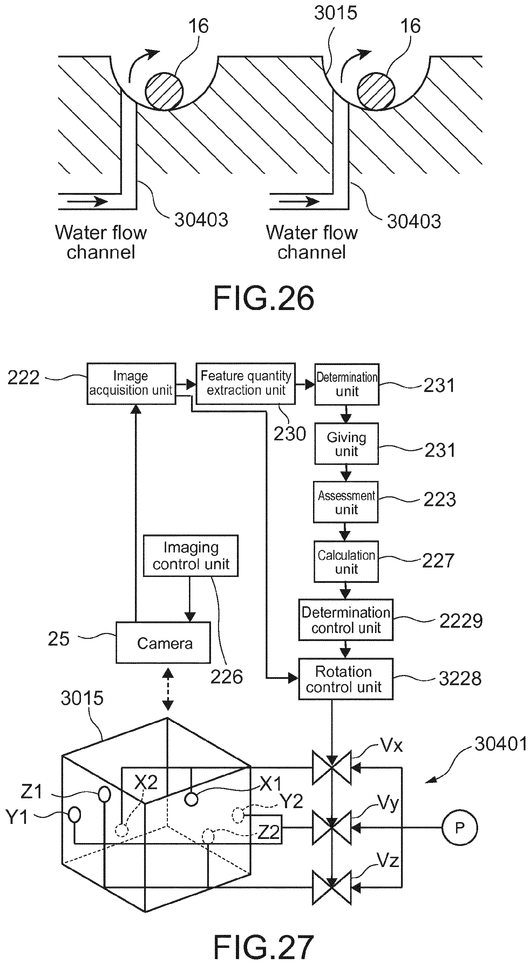

An observation system in the fifth embodiment will be described with reference to Figs. 24 to 27. Fig. 24 is a schematic diagram showing a configuration of the observation system. Fig. 25 is a block diagram showing a configuration of the observation system. Fig. 26 is an enlarged partial diagram of vicinities of the storage portions of the culture container. Fig. 27 is a diagram showing a configuration of a vicinity of the rotation unit of the observation system.

An observation system 3002 includes an observation apparatus 3021, an information processing apparatus 3022, the displaying apparatus 23, and the input apparatus 29. The observation illumination apparatus 24, the camera 25 serving as the imaging unit, the temperature/humidity/gas control unit 26, the stage 27, and a culture container 3040 including the rotation mechanism are arranged inside the observation apparatus 3021.

The culture container 3040 including the rotation mechanism includes a rotation unit 30401 serving as the rotation mechanism, storage portions 3015 that store the fertile ova 16, and micro flow channels (water flow channels) 30403. A micro flow channel control unit 30402 controls the rotation unit 30401. The rotation unit 30401 generates a flow in the culture medium 18 in the storage portions 3015 that store the fertile ova 16 to thereby rotate the fertile ova 16. The micro flow channel control unit 30402 controls ejection of fluid into the storage portions 3015 and generates a flow the culture medium 18 by ejecting the fluid. The water flow channel 30403 is a flow channel which is connected to each storage portion 3015 and through which fluid for supplying fluid into each storage portion 3015 flows.

As in the storage portions 15 of the culture container 1 according to each of the above-mentioned embodiments, the storage portion 3015 is capable of storing liquid and storing a single cell in the liquid while maintaining the single cell at a fixed position. The "liquid" is typically a culture medium suitable for culturing cells. Here, the "liquid" will be referred to as a culture medium.

The rotation unit 30401 includes a pump P, an X-axis-rotation valve Vx, a Y-axis-rotation valve Vy, a Z-axis-rotation valve Vz, a first X-axis ejection port X1 (first output port), a second X-axis ejection port X2 (second output port), a first Y-axis ejection port Y1 (first output port), a second Y-axis ejection port Y2 (second output port), a first Z-axis ejection port Z1 (first output port), and a second Z-axis ejection port Z2 (second output port). Here, the X-axis, the Y-axis, and the Z-axis mean three axes orthogonal to one another. The X-axis, the Y-axis, and the Z-axis do not mean horizontal or vertical directions.

The first X-axis ejection port X1, the second X-axis ejection port X2, the first Y-axis ejection port Y1, the second Y-axis ejection port Y2, the first Z-axis ejection port Z1, and the second Z-axis ejection port Z2 are formed in an inner wall surface of the storage portion 3015 (in a case where a plurality of storage portions 3015 is provided, the respective ejection ports are inherently formed with respect to each of all the storage portions 3015). The first X-axis ejection port X1, the second X-axis ejection port X2, the first Y-axis ejection port Y1, the second Y-axis ejection port Y2, the first Z-axis ejection port Z1, and the second Z-axis ejection port Z2 respectively eject (inject) fluid into the culture medium in the storage portion 3015 to thereby generate a flow in the culture medium in the storage portion 3015. The "liquid" is typically the same liquid as the culture medium in the storage portion 3015. Alternatively, the "liquid" is may be liquid or gas different from the culture medium in the storage portion 3015.

The pump P connects to each of the first X-axis ejection port X1, the second X-axis ejection port X2, the first Y-axis ejection port Y1, the second Y-axis ejection port Y2, the first Z-axis ejection port Z1, and the second Z-axis ejection port Z2 via a flow channel and supplies the culture medium into those ejection ports. A part of each flow channel (a part on the ejection port side, not on the pump side) is formed in the wall surface of the storage portion 3015 (in a case where a plurality of storage portions 3015 is provided, the respective flow channels are inherently formed with respect to each of all the storage portions 3015).

The flow channel that connects the pump P to the first X-axis ejection port X1 and the second X-axis ejection port X2 is provided with the X-axis-rotation valve Vx. The flow channel that connects the pump P to the first Y-axis ejection port Y1 and the second Y-axis ejection port Y2 is provided with the Y-axis-rotation valve Vy. The flow channel that connects the pump P to the first Z-axis ejection port Z1 and the second Z-axis ejection port Z2 is provided with the Z-axis-rotation valve Vz.

The micro flow channel control unit 30402 controls an ejection rate and an amount of ejection of the culture medium ejected through the respective ejection ports of the rotation unit 30401 on the basis of a control signal sent from a rotation control unit 3228 to be described later.

The information processing apparatus 3022 includes the image acquisition unit 222, the feature quantity extraction unit 230, the determination unit 231, the giving unit 232, the assessment unit 223, the storage unit 224, the display control unit 225, the imaging control unit 226, the calculation unit 227, the rotation control unit 3228, and the determination control unit 2229.

The information processing apparatus 3022 controls operations of the respective blocks in the observation system 3002. The image acquisition unit 222, the feature quantity extraction unit 230, the determination unit 231, the giving unit 232, the assessment unit 223, the storage unit 224, the display control unit 225, the imaging control unit 226, the calculation unit 227, the rotation control unit 3228, and the determination control unit 2229, which are the functional blocks of the information processing apparatus 3022, are realized in such a manner that the CPU loads a program stored in the ROM which is an example of a non-transitory computer-readable recording medium into the RAM and executes the loaded program. Then, those functional blocks execute the image acquisition method according to the present technology and the assessment method.

The rotation control unit 3228 receives a driving signal from the determination control unit 2229 and sends a control signal to the rotation apparatus 340 so as to activate the rotation unit 30401 at the time of imaging of the fertile ova 16. The rotation control unit 3228 is capable of controlling the direction of rotation and the amount of rotation of the fertile ovum 16 such that images of a desired fertile ova 16 can be obtained.

The rotation control unit 3228 receives a driving signal from the determination control unit 2229 and sends a control signal to the micro flow channel control unit 30402 so as to activate the rotation unit 30401.

When the operation of the rotation unit 30401 is started on the basis of a control signal sent from the rotation control unit 3228 to the micro flow channel control unit 30402, fluid is ejected into the storage portion 3015, a flow is generated in the culture medium 18 in the storage portion 2015, and the fertile ovum 16 is rotated.

The rotation control unit 3228 is capable of controlling the direction of rotation and the amount of rotation of the fertile ovum 16 such that images of a desired fertile ova 16 can be obtained.

Here, regarding the fertile ovum 16 on the growth stage after the blastocyst 1609, the shape and the position of the ICM 161 in the fertile ovum 16 differ in a manner that depends on the observation directions. For example, in order to acquire an image in which the ICM 161 is positioned at the center of the fertile ovum 16, the image in which the ICM 161 is positioned at the center of the fertile ovum 16 can be acquired by rotating the fertile ovum 16 in the following manner.

The rotation control unit 3228 detects the shape and position of the ICM 161 by image recognition such as edge detection on the basis of the image of the fertile ovum 16 imaged just before the fertile ovum 16 is rotated. Based on it, the rotation control unit 3228 calculates a direction of rotation and an amount of rotation of the fertile ovum 16 in order to cause the ICM 161 to be positioned at the center of the fertile ova 16. The rotation unit 20401 only needs to rotate the fertile ova 16 in accordance with the calculated direction of rotation and amount of rotation.

The micro flow channel control unit 30402 individually controls, on the basis of a control signal from the rotation control unit 3228, a flow of the culture medium which is generated by each ejection port X1, X2, Y1, Y2, Z1, or Z2 of the rotation unit 30401 to thereby control the direction of rotation and the amount of rotation of the fertile ovum 16. Specifically, the micro flow channel control unit 30402 controls open/close of the X-axis-rotation valve Vx on the basis of the control signal from the rotation control unit 3228 to thereby control the ejection rate and the amount of ejection of the culture medium ejected through the first X-axis ejection port X1 and the second X-axis ejection port X2. The micro flow channel control unit 30402 controls open/close of the Y-axis-rotation valve Vy on the basis of the control signal from the rotation control unit 3228 to thereby control the ejection rate and the amount of ejection of the culture medium ejected through the first Y-axis ejection port Y1 and the second Y-axis ejection port Y2. The micro flow channel control unit 30402 controls open/close of the Z-axis-rotation valve Vz on the basis of the control signal from the rotation control unit 3228 to thereby control the ejection rate and the amount of ejection of the culture medium ejected through the first Z-axis ejection port Z1 and the second Z-axis ejection port Z2.

As described above, the fluid may be ejected (injected) into the culture medium in the storage portion of the culture container to thereby generate a flow in the culture medium in the storage portion and rotate the fertile ovum 16.

### (Sixth Embodiment)

Next, an observation system having a configuration different from that of the observation system in each of the above-mentioned embodiments will be described as a sixth embodiment. It should be noted that the configuration of the observation system is not limited thereto.

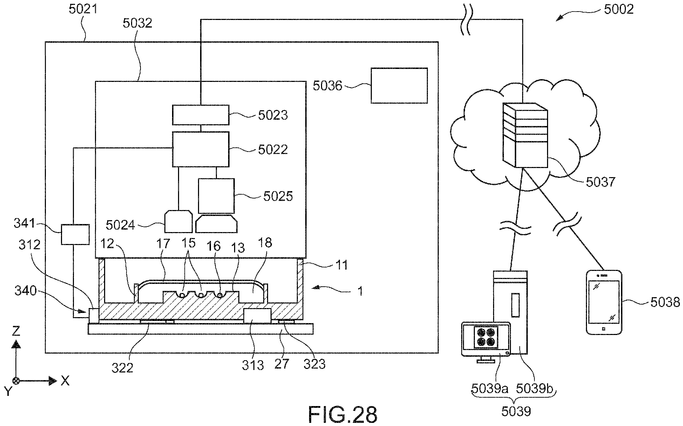

In each of the above-mentioned embodiments, the culture container 1 and the camera 25 are installed inside the observation apparatus 21 that observes the fertile ova and the information processing apparatus 22 is installed outside the observation apparatus 21, though not limited thereto. For example, as shown in Fig. 28, an information processing apparatus 5022 may be installed in an observation apparatus 5021.

Fig. 28 shows a configuration of an observation system 5002 according to a sixth embodiment. As shown in Fig. 28, the observation system 5002 includes the observation apparatus 5021. The observation apparatus 5021 is connectable to a cloud server 5037 via a network. Furthermore, a portable terminal 5038 and a personal computer 5039 which are to be displaying apparatuses are each connectable to the cloud server 5037 via the network. An integrated camera-information processing apparatus unit 5032 and a temperature/humidity/gas control unit 5036 are installed in the observation apparatus 5021 and the culture container 1 is stored.

The integrated camera-information processing apparatus unit 5032 includes a camera 5025 as an imaging unit, an observation illumination apparatus 5024, the information processing apparatus 5022, and a communication unit 5023. In this embodiment, the observation illumination apparatus 5024 for light to be radiated to the fertile ova 16 is arranged above the culture container 1, not below the culture container 1.

The observation illumination apparatus 5024 emits light to be radiated to the culture container 1 when the camera 5025 images the fertile ova 16 in the culture container 1. The camera 5025 images the fertile ova 16 in the culture container 1.

The information processing apparatus described in each of the above-mentioned embodiments is applicable to the information processing apparatus 5022. The information processing apparatus 5022 acquires images of the fertile ovum 16 on each growth stage. Furthermore, with respect to the fertile ovum 16 on the growth stage after the blastocyst, the information processing apparatus 5022 acquires images of the fertile ovum 16 imaged at a plurality of angles by imaging the fertile ovum 16 in the state in which the fertile ovum 16 is rotated, for example. The information processing apparatus 5022 outputs, to the cloud server 5037 via the communication unit 5023 and the network, the image of the fertile ovum 16 and data signals of position information of the storage portion in which the fertile ovum 16 is stored, date and time of imaging, an imaging condition, a second feature quantity of the fertile ovum, a growth stage code, an observation direction classification code, and the like (hereinafter, referred to as data signals associated with the fertile ovum 16), which are associated with this image.

The temperature/humidity/gas control unit 5036 controls temperature, humidity, and gas inside the observation apparatus 5021. The temperature/humidity/gas control unit 5036 makes an environment suitable for culturing the fertile ova 16.

The communication unit 5023 receives the data signals associated with the fertile ovum 16 from the information processing apparatus 5022 and outputs the received data signals to the cloud server 5037 via the network.

The cloud server 5037 stores the data signals associated with the fertile ovum 16. The personal computer 5039 including a display unit 5039a and an information processing unit 5039b or the portable terminal 5038 receives and displays the data signals associated with the fertile ovum 16 from the cloud server 5037 via the network in accordance with a user's operation for operating them.

Although the respective embodiments of the present technology have been described above, the present technology is not limited only to the above-mentioned embodiments and various modifications can be made without departing from the gist of the present technology as a matter of course.

It should be noted that the present technology may also take the following configurations.
(1) An information processing apparatus, including:
   a storage unit that pre-stores a plurality of first images, the plurality of first images being obtained by rotating and imaging a comparative cell;
   an image acquisition unit that acquires a plurality of second images, the plurality of second images being obtained by rotating and imaging a cell that is an object to be assessed; and
   an assessment unit that assesses the cell that is the object to be assessed on the basis of a result of comparison of the first image with the second image.
(2) The information processing apparatus according to (1), in which
   the assessment unit assesses the cell that is the object to be assessed on the basis of a result of comparison of a first feature quantity extracted from the first image with a second feature quantity extracted from the second image.
(3) The information processing apparatus according to (1) or (2), in which
   the storage unit stores an assessment result of the first image which is associated with the first image, further including:
   a determination unit that compares the first image with the second image and determines whether or not the first image is identical to the second image; and
   a giving unit that gives the second image the assessment result associated with the first image determined to be identical to the second image in a case where the determination unit determines that the first image is identical to the second image, in which
   the assessment unit considers a most given assessment result of assessment results respectively given to the plurality of second images as an assessment of the cell that is the object to be assessed.
(4) The information processing apparatus according to (3), further including:
   a calculation unit that calculates an occupancy rate of the most given assessment result of the assessment results respectively given to the plurality of second images; and
   a determination control unit that
      determines whether or not a numerical value of the rate calculated by the calculation unit is stable, and
      controls the determination unit to compare the first image with the second image until the determination control unit determines that the numerical value of the rate is stable.
(5) The information processing apparatus according to (4), further including
   a rotation control unit that controls a rotation mechanism that rotates the cell.
(6) The information processing apparatus according to (5), in which
   the rotation control unit controls, in a case where the determination control unit determines that the numerical value of the rate is stable, the rotation mechanism to rotate the cell that is the object to be assessed.
(7) The information processing apparatus according to any one of (1) to (6), in which
   the assessment unit assesses a growth stage of the cell that is the object to be assessed.
(8) The information processing apparatus according to any one of (1) to (7), in which
   the assessment unit assesses quality of the cell that is the object to be assessed.
(9) An information processing system, including:
   a culture container including a plurality of storage portions in which a cell is stored;
   an imaging unit that images the cell;
   an image acquisition unit that acquires an image of the cell imaged by the imaging unit;
   a rotation mechanism that rotates the cell in one of the storage portions;
   a storage unit that pre-stores a first image acquired in such a manner that the imaging unit images the comparative cell rotated by the rotation mechanism;
   an image acquisition unit that acquires a plurality of second images, the plurality of second images being obtained in such a manner that the imaging unit images the cell that is an object to be assessed after the rotation mechanism rotates the cell that is the object to be assessed; and
   an assessment unit that assesses the cell that is the object to be assessed on the basis of a result of comparison of the first image with the second image.
(10) An information processing method, including:
   acquiring a plurality of second images by rotating and imaging a cell that is an object to be assessed;
   comparing a plurality of first images with each of the second images, the plurality of first images being acquired in advance by rotating and imaging a comparative cell; and
   assessing the cell that is the object to be assessed on the basis of the result of comparison.
(11) A program that causes a computer to execute:
   a step of acquiring a plurality of second images by rotating and imaging a cell that is an object to be assessed;
   a step of comparing a plurality of first images with each of the second images, the plurality of first images being acquired in advance by rotating and imaging a comparative cell; and
   a step of assessing the cell that is the object to be assessed on the basis of the result of comparison.

### Reference Signs List

1, 3040 culture container
2, 1002, 2002, 3002, 5002 observation system
(information processing system)
15, 3015 storage portion
16 fertile ovum (cell)
22, 1022, 2022, 3022, 5022 information processing apparatus
25, 5025 camera (imaging unit)
222 image acquisition unit
223 assessment unit
224 storage unit
2241 learning database
2242 analysis-result database
227 calculation unit
228, 2228, 3228 rotation control unit
229, 1229, 2229 determination control unit
231 determination unit
340 rotation apparatus (rotation mechanism)
30401 rotation unit (rotation mechanism)

## Claims

1. An information processing apparatus, comprising:
a storage unit that pre-stores a plurality of first images, the plurality of first images being obtained by rotating and imaging a comparative cell;
an image acquisition unit that acquires a plurality of second images, the plurality of second images being obtained by rotating and imaging a cell that is an object to be assessed; and
an assessment unit that assesses the cell that is the object to be assessed on a basis of a result of comparison of the first image with the second image.

2. The information processing apparatus according to claim 1, wherein
the assessment unit assesses the cell that is the object to be assessed on a basis of a result of comparison of a first feature quantity extracted from the first image with a second feature quantity extracted from the second image.

3. The information processing apparatus according to claim 2, wherein
the storage unit stores an assessment result of the first image which is associated with the first image, further comprising:
a determination unit that compares the first image with the second image and determines whether or not the first image is identical to the second image; and
a giving unit that gives the second image the assessment result associated with the first image determined to be identical to the second image in a case where the determination unit determines that the first image is identical to the second image, wherein
the assessment unit considers a most given assessment result of assessment results respectively given to the plurality of second images as an assessment of the cell that is the object to be assessed.

4. The information processing apparatus according to claim 3, further comprising:
a calculation unit that calculates an occupancy rate of the most given assessment result of the assessment results respectively given to the plurality of second images; and
a determination control unit that
determines whether or not a numerical value of the rate calculated by the calculation unit is stable, and
controls the determination unit to compare the first image with the second image until the determination control unit determines that the numerical value of the rate is stable.

5. The information processing apparatus according to claim 4, further comprising
a rotation control unit that controls a rotation mechanism that rotates the cell.

6. The information processing apparatus according to claim 5, wherein
the rotation control unit controls, in a case where the determination control unit determines that the numerical value of the rate is stable, the rotation mechanism to rotate the cell that is the object to be assessed.

7. The information processing apparatus according to claim 6, wherein
the assessment unit assesses a growth stage of the cell that is the object to be assessed.

8. The information processing apparatus according to claim 7, wherein
the assessment unit assesses quality of the cell that is the object to be assessed.

9. An information processing system, comprising:
a culture container including a plurality of storage portions in which a cell is stored;
an imaging unit that images the cell;
an image acquisition unit that acquires an image of the cell imaged by the imaging unit;
a rotation mechanism that rotates the cell in one of the storage portions;
a storage unit that pre-stores a first image acquired in such a manner that the imaging unit images the comparative cell rotated by the rotation mechanism;
an image acquisition unit that acquires a plurality of second images, the plurality of second images being obtained in such a manner that the imaging unit images the cell that is an object to be assessed after the rotation mechanism rotates the cell that is the object to be assessed; and
an assessment unit that assesses the cell that is the object to be assessed on a basis of a result of comparison of the first image with the second image.

10. An information processing method, comprising:
acquiring a plurality of second images by rotating and imaging a cell that is an object to be assessed;
comparing a plurality of first images with each of the second images, the plurality of first images being acquired in advance by rotating and imaging a comparative cell; and
assessing the cell that is the object to be assessed on a basis of the result of comparison.

11. A program that causes a computer to execute:
a step of acquiring a plurality of second images by rotating and imaging a cell that is an object to be assessed;
a step of comparing a plurality of first images with each of the second images, the plurality of first images being acquired in advance by rotating and imaging a comparative cell; and
a step of assessing the cell that is the object to be assessed on a basis of the result of comparison.
